# EUROPEAN PATENT SPECIFICATION

(11) **EP 4 225 173 B1**
(45) Date of publication and mention of the grant of the patent: **12.02.2025**
(21) Application number: 21802419.8
(22) Date of filing: 11.10.2021
(51) Int. Cl.: A61B 17/34, A61M 25/01, A61M 25/00

(54) **A DEVICE FOR ENDOSCOPICALLY DELIVERING A THERAPEUTIC SUBSTANCE**
VORRICHTUNG ZUR ENDOSKOPISCHEN ABGABE EINER THERAPEUTISCHEN SUBSTANZ
DISPOSITIF D'ADMINISTRATION ENDOSCOPIQUE D'UNE SUBSTANCE THÉRAPEUTIQUE

(30) Priority: 09.10.2020 IT 202000023836
(43) Date of publication of application: 16.08.2023
(73) Proprietor: Scuola Superiore di Studi Universitari e di Perfezionamento Sant'Anna, 56127 Pisa (IT)
(72) Inventor: RICOTTI, Leonardo, 56037 Peccioli (PI) (IT); VANNOZZI, Lorenzo, 56024 Pisa (IT); MAZZOCCHI, Tommaso, 51016 Montecatini Terme (PT) (IT); SILIBERTO, Alessio, 74023 Grottaglie (TA) (IT); GUARNERA, Daniele, 95121 Catania (IT); RESTAINO, Francesco Rocco, 85100 Potenza (PZ) (IT)
(74) Representative: ABM Agenzia Brevetti & Marchi
(86) International application number: PCT/IB2021/059279
(87) International publication number: WO 2022/074632

(56) References cited:
- KR-A- 20130 012 700
- US-A- 4 353 358
- US-A- 5 167 623
- US-A- 5 250 038
- US-A- 5 285 795
- US-A- 5 378 230
- US-A- 5 624 397
- US-A- 6 126 649
- US-A1- 2007 250 111
- US-A1- 2013 190 562
- US-A1- 2015 025 311
- US-A1- 2016 228 136

## Description

### Field of the invention

The present invention relates to an endoscopic extrusion device, i.e., a device for delivering a fluid therapeutic substance in a status in an articular cavity.

In particular, the invention relates to an arthroscopic extrusion device suitable for delivering such a therapeutic substance into an articular cavity for a minimally invasive treatment of an injured articular cartilage, in particular, in mild and severe osteoarthritis.

### Technical front and technical problems

As well known, endoscopic surgery techniques offer important advantages over open surgery, in particular they are less invasive to the tissues surrounding the operation site, make it possible to shorten the treatment time and therefore cause less discomfort to the patients, shorten the length of stay in hospital and reduce the treatment costs.

These advantages are potentially important for treating an articular tissue, in particular an injured articular cartilage, typically in a phase III and IV osteoarthritis.

The partial surface cartilage wear in phase III osteoarthritis is normally treated by hyaluronic acid intra-articular injection and by chondrocyte-protective substances, and/or by autologous chondrocyte or stem cell transplantation. The latter two techniques consist in taking cartilage cells from another bone region of the patient, *in vitro* cultivating the cells during a few weeks and implanting them in the patient once they have differentiated. The cartilage lesion must be preliminarily cleaned and coated with a periosteum in which a hole is provided for injecting and retaining a solution of the cultivated cells, so that the latter form new cartilage *in situ.* This requires several treatments, and a long time is needed for both the cell cultivation and the operation itself. Moreover, the step cannot be carried arthroscopically, on the contrary, open surgery is necessary.

Phase IV osteoarthritis, when not so severe to require an articular prosthesis, are often treated arthroscopically by mosaicoplasty. This technique consists in a cartilage transplantation. A portion of cartilage is taken from a less worn region of the same joint and is then inserted under pressure in the damaged region. The limited availability of transplantable material limits this technique to small lesions. Moreover, small joints such as finger or spinal joints cannot be treated by this technique. Another drawback of this technique is that the transplantation is less effective in patients older than 40-50 years, because the cartilage proliferative capacity decreases with age.

Therefore, it would be desirable to carry out minimally invasive surgical treatments for treating damaged articular tissues, in order to join the above summarized general advantages of the endoscopic techniques, with such more specific benefits as avoiding multiple treatment in the time, preventing the cartilage replacement tissue detachment from the application site, overcoming age limitations and making autologous withdrawal unnecessary.

Several devices for extruding a biologic substance are known for endoscopic use, as described in WO2018078130, US2019008998, CN210174208, CN206528075U. These documents relate to devices for extruding biologic substances at an operation site in the body of a patient, comprising a cannula to be introduced into the body through an endoscopic access, and a device for feeding and pushing the biologic substances to be extruded at a predetermined operation site.

Moreover, if multiple biologic substances must be stratified, for example, to form a primer layers or coaxial layers, coaxial extruder are known comprising two coaxial annular ducts.

In any case, it is necessary that the biologic substances are uniformly distributed in a predetermined area, in directions laying in an angle centred about a predetermined middle direction. In the cited prior art, this is obtained by forming an endoscopic access in said middle direction, and leaving the surgeon the task of directing the extrusion mouth of the cannula within this angle during the release. However, the prior art devices provide stiff or even flexible but not orientable cannulas, which complicates such a procedure. This can be the cause of an uneven distribution of the biologic substances.

US 2015/025311 A1 discloses a device for endoscopically delivering at least one therapeutic substance into a knee joint.

Briefly, despite the important search efforts in this field, till now no device has been practically available to extrude *in situ* a biologic and/or therapeutic material fulfilling the above-mentioned requirements.

### Summary of the invention

It is therefore an object of the present invention to provide a device that allows a minimally invasive delivery of therapeutic substances, in particular therapeutic substances for regenerating the cartilage, into a small articular cavity or into an articular cavity that is difficult to attain by the prior art devices, such as a knee or elbow articular cavity.

It is also an object of the invention to provide such a device that assists a uniform distribution of said therapeutic substances in such articular cavity.

It is also an object of the invention to provide such a device that encounter a negligible resistance while travelling through the biological material between the body access site and the operation site.

It is also an object of the invention to provide such a disposable device and that, to this purpose, is easy and cheap to manufacture.

These and other objects are achieved by an arthroscopic delivery device as defined in claim 1. Advantageous exemplary embodiments of the device are defined by the dependent claims.

According to an aspect of the invention, a device for endoscopically delivering at least one therapeutic substance comprises:
- a cannula defining at least one extrusion channel and comprising:
   - a proximal portion;
   - a flexible distal portion having an extrusion mouth and
   - a substantially rigid intermediate portion between the flexible distal portion and the proximal portion,
   wherein the proximal portion is configured to feed and push a therapeutic substance into the extrusion channel, so that the therapeutic substance can pass through the extrusion channel flowing through the intermediate portion and through the flexible distal portion and can then exit from the extrusion mouth,
- at least one actuation cable having:
   - a proximal end operable at the proximal portion of the cannula;
   - a distal end connected to the flexible distal portion of the cannula at the extrusion mouth, and configured to pull the flexible distal portion with a force transversal to the extrusion channel, bringing the flexible distal portion from an undeformed conformation to a deformed conformation,
wherein the substantially rigid intermediate portion has a distal rigid edge from which the flexible distal portion protrudes, and wherein the distal rigid edge has a passageway exit for the actuation cable, said distal edge and said passageway exit arranged in such a way that only the flexible distal portion of the cannula can be brought, by the actuation cable, from the undeformed conformation to the deformed conformation while the intermediate portion is always undeformed.

The actuation cable allows a surgeon to accurately control the direction the flexible distal portion of the cannula, which is not possible by the known tools, such as those of the cited prior art. This way, the surgeon can modulate the bending of the flexible distal portion of the cannula and, by suitably orienting the cannula, can easily select the extrusion direction of the therapeutic substance according to the operation requirements.

This way, moreover, the actuation cable is maintained close to the flexible distal portion of the cannula, which allows a more precise bending of the same. Furthermore, the above-described arrangement makes it easier to introduce the cannula into the patient's body, preventing the actuation cable from getting caught through the various biological material that is present between the body access site and the operation site. Moreover, the guide element provides circumferential and/or radial constraint forces acting on the cannula, while the surgeon operates the tie-member, i.e., the actuation cable.

In an exemplary embodiment, the distal rigid edge can comprise at least one guide element for the actuation cable or for each actuation cable, the guide element or the guide elements arranged along an outer surface of the cannula, the passageway being made within the guide element, through which the actuation cable or a respective actuation cable is slidingly arranged.

The device also makes it possible to attain particularly extended areas of an operation site, due to the flexibility of the end portion. In particular, the device allows an easy and minimally invasive delivery of the therapeutic substance on articular cavity portions that would be difficult to attain by the current instruments, for instance, the knee or elbow articular cavities, and the like.

In a preferred exemplary embodiment of the invention, a flexible rod extends from the distal rigid edge of the intermediate portion, said rod comprising a plurality of ring portions that surround the flexible distal portion of the cannula, wherein the plurality of ring portions comprises a terminal ring to which the distal end of the actuation cable is connected. This way, by pulling the actuation cable, the flexible rod bends and the ring portions follow and guide the flexible distal portion of the cannula from the undeformed conformation to the deformed conformation, the actuation cable arranged to slide within the ring portions. This way, the distal portion of the cannula has a smaller radial overall size, in particular, at the extrusion mouth, which makes it easier to move the cannula between the body access site and the operation site.

In particular, the distal portion and the intermediate portion of the cannula comprise:
- a common tubular core element made of a flexible material, the tubular core element defining the extrusion channel;
- a common shell element enclosing the tubular core element,
wherein the shell element comprises the distal rigid edge and the flexible rod.

Advantageously, the flexible rod comprises an elongated support portion extending parallel to the longitudinal axis of the cannula from a sector of the distal edge, the ring portions extending transversally to the elongated support portion between two opposite lateral sides thereof.

Advantageously, the flexible distal portion of the cannula is resiliently compliant. This way, by releasing the actuation cable after stretching it, the flexible distal portion returns from the deformed conformation to the undeformed conformation, or also to an intermediate conformation between undeformed conformation And the deformed conformation.

In a possible exemplary embodiment, the at least one actuation cable is a first actuation cable, and a second actuation cable is provided configured to pull the flexible distal portion in an opposite direction with respect to the first actuation cable. This way, the first actuation cable and the second actuation cable can selectively work as an agonist actuation cable and an antagonist actuation cable, and vice-versa. This allows a more stable positioning of the extrusion mouth of the cannula in a predetermined delivery position.

In a possible exemplary embodiment, at least one further actuation cable is provided comprising a distal end connected to the flexible distal portion at the extrusion mouth, and configured to pull the flexible distal portion bring it from the undeformed conformation to a further deformed conformation in a bend plane different from a plane of the deformed conformation defined by the axis of the cannula and by the axis of the previously mentioned actuation cable. This way, the flexible distal portion of the cannula can be bent in a plane that is distinct from the plane defined by the axis of the cannula, i.e., by the axis of the proximal and intermediate rigid portions thereof, and by the axis of the first actuation cable. More in detail, it is possible this way to bend the flexible distal portion along any bending plane of the plurality of planes passing through the axis of the cannula, by suitably combining the pulling force exerted on the first actuation cable and the pulling force exerted on the further actuation cable. This makes less necessary for the surgeon to rotate the cannula about its own axis in order to bring the extrusion mouth to a predetermined position about the axis of the cannula.

Even in this case, obviously, a respective second actuation cable can be provided for the first and for the further actuation cable in a position diametrically opposite with respect to the cannula, the second actuation cable arranged to work as an antagonist cable or as an agonist cable in opposition to the first and to the further actuation cable.

In an exemplary embodiment, the extrusion channel is configured as a central extrusion channel and an annular extrusion channel coaxial to each other, and the proximal portion is configured to feed and push a first therapeutic substance into the central extrusion channel and a second therapeutic substance into the annular extrusion channel, and to deliver the therapeutic substances through the extrusion mouth with the flexible distal portion in the deformed conformation.

In an alternative exemplary embodiment, the extrusion channel is configured as a central extrusion channel, a first annular extrusion channel and a second annular extrusion channel coaxial to one another, and the proximal portion of the cannula is configured to feed and push a first therapeutic substance into the first annular extrusion channel and a second therapeutic substance into the second annular extrusion channel, or a first therapeutic substance into the central extrusion channel, a second therapeutic substance into the first annular extrusion channel and a third therapeutic substance into the second annular extrusion channel, and to deliver the therapeutic substances through the extrusion mouth with the flexible distal portion in the deformed conformation.

In another alternative exemplary embodiment, the extrusion channel is configured as a first and a second extrusion channel parallel to each other, and the proximal portion is configured to feed and push a first and a second therapeutic substance into the first and second extrusion channel, respectively, and to deliver the therapeutic substances through the extrusion mouth with the flexible distal portion in the deformed conformation.

The devices according to each of the last three exemplary embodiments allow to extrude at the same time two or three different therapeutic substances, each of them having a specific function, through respective extrusion channels. In these cases, the different therapeutic substances to be extruded are stored within different compartments of the device.

More in detail, in case of the device having a central extrusion channel and two annular extrusion channels coaxial to one another, the proximal portion is configured to extrude layers of therapeutic substances commonly indicated as "core" and "shell" in the two inner extrusion channels. The core contains cells for repairing the damaged portion of the cartilage in order to build up new tissue and a biomaterial to create an environment suitable fr the cells to proliferate and differentiate. The shell is also formed by hydrogels as well, in order to protect the core from both the shear stresses generated by the extrusion, and the free radicals that can be formed during a possible photopolymerization process to stabilize the extruded material. The proximal portion is also configured to use the outer layer to extrude a "primer" in order to assist the adhesion of the two inner layers, thus forming a core-shell structure at the site lesion site, which can consolidate even after the extrusion.

In comparison with the prior art, in which such a fluid substance as a surgical glue is delivered by different tools and in times different than the surgical operation, the invention provides a single device for depositing a plurality of therapeutic substances at the same time, thus reducing the time, the costs and the disadvantages associated with the treatment.

More in particular, the device according to this exemplary embodiment allows treating the damaged cartilage of osteoarthritis-affected joints by arthroscopically extruding in situ a combination of materials in a single surgical step. Therefore, multiple and long-lasting operations can be avoided, as currently required by conventional autologous chondrocyte and/or staminal cell implant techniques to treat joints with phase III osteoarthritis, and also the open surgery required to threat phase IV osteoarthritis extended lesions.

Advantageously, at the extrusion mouth a rigid spacer element is provided, to which it the distal end of the actuation cable is connected, and the rigid spacer element is configured to engage with and keep open the extrusion mouth when the flexible distal portion moves from the undeformed conformation to the deformed conformation. In other words, the walls of the central extrusion channel and of the annular channel or channels are constrained at predetermined points so as to maintain the extrusion channels concentrical to one another even in the deformed conformation.

The flexible distal portion can be made of a biocompatible polymeric material that also has such features as high deformability and flexibility, and low tendency to kinking.

For instance, the flexible distal portion of the cannula can be made of a heat-shrinking material, so as to anchor the flexible distal portion to the intermediate portion by introducing the intermediate portion into the distal portion and by performing a subsequent thermal cycle. This makes it possible to obtain a particularly strong connection between the flexible distal portion and the intermediate portion of the cannula.

As an alternative, the wall of the extrusion channel, i.e., the walls of the cylindrical extrusion channels of the flexible distal portion are made as a composite material in which a preferably helical compression spring having a predetermined length, a predetermined diameter and a predetermined wire thickness is incorporated in a polymeric tubular matrix. This structure can be obtained by such a prior art technique as dip-coating, in which the spring is immersed into a polymeric solution, then it is extracted and caused to dry or to crosslink. This way, composite walls are obtained that are particularly thin, but that are much more rigid and a radially resistant than they would be if they were exclusively formed by a polymeric material. Such composite walls are substantially free from occlusion issues during their use and have a good elastic recovery to their initial conformation after the use.

These composite structures, and the relative production process, can also be used also in the case of a flexible distal portion having concentric annular extrusion channels, and can be used for the walls of all the extrusion channels. The composite walls obtained this way, can be singularly connected at the distal end of the intermediate portion of the cannula and can preferably be connected to one another, in such a way to maintain the concentricity during the bending.

As an alternative, the flexible distal portion of the cannula can be connected to the intermediate portion of the cannula by a fixed joint, wherein a proximal part of the flexible distal portion and a distal part of the intermediate portion are introduced into each other forming a force-fitting connection.

In particular, the cannula can comprise a convergence chamber configured to convey two or three materials into the proximal part, and two or three concentric extrusion channels configured to keep the materials separate form one another during the extrusion through the extrusion mouth.

Preferably, the flexible distal portion has a length set between 1 mm and 10 mm, and is configured in such a way that the deformed conformation has the shape of an arc.

Concerning the size, the diameter of the cannula, possibly containing a plurality of extrusion channels, is advantageously set between 1 mm and 15 mm, preferably between 2 mm and 8 mm, while its length is preferably set between 100 and 140 mm.

In the case of a cannula including three concentric extrusion channels at least in the flexible distal portion, the diameter of the central extrusion channel is advantageously chosen in such a way to limit the shear stress acting on the therapeutic substance while being extruded therethrough. This is useful when the therapeutic substance comprises cells in culture. The diameter of the central extrusion channel is preferably set between 1 and 2 mm. This way, in normal extrusion conditions, the shear stress acting on the material being extruded is not higher than 2 kPa.

The walls of the single extrusion channels have thickness preferably set between 100 and 500 µm, in particular between 200 and 300 µm, according to the production process.

In any case, the size of the flexible distal portion is preferably selected in such a way to limit the extrusion pressure. Considering an extrusion channel wall thickness of about 200-300 µm, the extrusion space of the internal annular extrusion channel inner ring can range between 200 and 500 µm. Similarly, the internal annular extrusion channel can have an extrusion space ranging between 200 and 500 µm.

The rigid part of the cannula, i.e., the distal and/or intermediate portion, can be made of different materials, for instance AISI 316L stainless steel. The production process can be based on additive manufacturing methods such as Powder Bed Fusion Selective Laser Sintering (SLS), Direct Melting Laser Sintering (DMLS) or Electron Beam Melting (EBM).

The materials of all the parts of the cannula are selected among the biocompatible materials that are also resistant to wear and corrosion, and that can be transformed by one of the manufacturing processes mentioned above in connection with the single portions.

Advantageously, the proximal portion is associated with a handpiece configured to allow a surgeon to manipulate the whole device.

According to another aspect of the invention, the extrusion channels are provided by the lumina of a multi-lumen tube housed within a shell portion surrounding the proximal and intermediate portions of the cannula, wherein the multi-lumen tube distally protrudes out of the shell portion to form at least one part of the flexible distal portion of the cannula. In a particular exemplary embodiment, also the passageways for respective actuation cables are provided by further lumina of the multi-lumen tube.

### Brief description of the drawings

The invention is shown hereafter by the description of some exemplary embodiments, exemplifying but not limitative, with reference to the attached drawings, in which:
- Fig. 1 is a diagrammatical perspective view of a device according to the invention;
- Figs. 2 and 3 are diagrammatical partial perspective views of the cannula of the device of Fig. 1 in an undeformed conformation and in a deformed configuration, respectively;
- Fig. 2A shows a distal fastening element for an actuation cable;
- Figs. 4-6 are diagrammatical perspective views of a device according to an exemplary embodiment of the invention, in which a possible embodiment of the actuation unit of the device is shown;
- Figs. 7 and 8 diagrammatically show lateral views of a motor of the actuation unit of Figs. 5 and 6, when the flexible distal portion is in the undeformed configuration of Fig. 2 and in the deformed configuration of Fig. 3, respectively;
- Fig. 9 is a diagrammatical perspective view of a device according to an exemplary embodiment of the invention, including two actuation cables each configured to selectively work as an agonist actuation cable and as an antagonist actuation cable, and optionally comprising an actuation unit as shown in Figs. 5 and 6;
- Fig. 10 is a diagrammatical perspective view of two motors of the actuation unit of the device of Fig. 9;
- Figs. 11, 12 and 15 are diagrammatical partial perspective views of the cannula of the device of Fig. 9 in an undeformed conformation, in a first deformed configuration and in a second deformed configuration, respectively;
- Figs. 13 and 14 are diagrammatical lateral views of one of the motors of the actuation unit of the device of Fig. 9 when the flexible distal portion is in the undeformed configuration of Fig. 11 and in the first deformed configuration of Fig. 12, respectively;
- Figs. 16 and 17 are diagrammatical lateral views of the other of the motors of the actuation unit of the device of Fig. 9 when the flexible distal portion is in the undeformed configuration of Fig. 11 and in the second deformed configuration of Fig. 15, respectively;
- Figs. 18 and 19 are cross sectional views of cannulas of devices according to exemplary embodiments of the invention in which two actuation cables and two couples of actuation cables are provide, respectively, for bringing the flexible distal portion of the cannula to deformed conformations lying on different planes;
- Fig. 20 is a diagrammatical partial perspective view of the cannula of a device in which the extrusion channel is configured as a central extrusion channel and an annular extrusion channel coaxial to each other;
- Fig. 21 is a diagrammatical perspective view of a device according to an exemplary embodiment of the invention, in which the extrusion channel is configured as a central extrusion channel and first and second annular extrusion channels coaxial to one another;
- Fig. 22 is a view of a detail of the device of Fig. 21 showing a distribution system of three therapeutic substances into respective extrusion channels of the cannula;
- Figs. 23, 24 and 26 are diagrammatical partial perspective views of the cannula of the device of Fig. 21 in an undeformed conformation, with a rigid spacer element removed (Fig. 23) and mounted (Figs. 24 and 26), respectively;
- Fig. 25 is a perspective view diagrammatical view of the rigid spacer element of Fig. 24;
- Figs. 27A-H are diagrammatical perspective views showing manufacturing steps of three concentric extrusion channels, i.e., the steps of mounting the walls of the three concentric extrusion channels of the device of Fig. 21;
- Fig. 28 is a diagrammatical partial perspective view of the cannula of a device in which the extrusion channel is configured as first and second extrusion channels parallel to each other;
- Fig. 29 is a diagrammatical partial perspective view of the cannula of a device according to an exemplary embodiment of the invention in which the extrusion channel comprises a laminar first extrusion channel and a laminar second extrusion channel parallel to each other;
- Figs. 30 and 31 are diagrammatical partial perspective views of cannulas of a device according to another aspect of the invention, in which the extrusion channels and the passageways for respective actuation cables are provided by the lumina of a multi-lumen tube that is housed within the proximal and intermediate portions of the cannula and protrudes therefrom to form the flexible distal portion;
- Fig. 32 is a cross sectional view of the cannula of a device according to a modification of the exemplary embodiment of Fig. 20, in which the section of the external extrusion channel extends along a 180° arc;
- Fig. 33 is a cross sectional view of the cannula of a device according to another aspect of the invention in which, in a modification of the embodiments of Figs. 20 and 32, both the extrusion channels and the passageways for respective actuation cables are provided by the lumina of a multi-lumen tube;
- Figs. 34 and 35 are diagrammatical perspective views from two different viewpoints of a device according to a preferred exemplary embodiment of the invention;
- Fig. 36 is an elevation front view of the device of Figs. 34 and 35;
- Figs. 37 and 38 are diagrammatical partial side views of the cannula of the device of Figs. 34 and 35 in an undeformed conformation and in a deformed configuration, respectively.

### Description of preferred exemplary embodiments

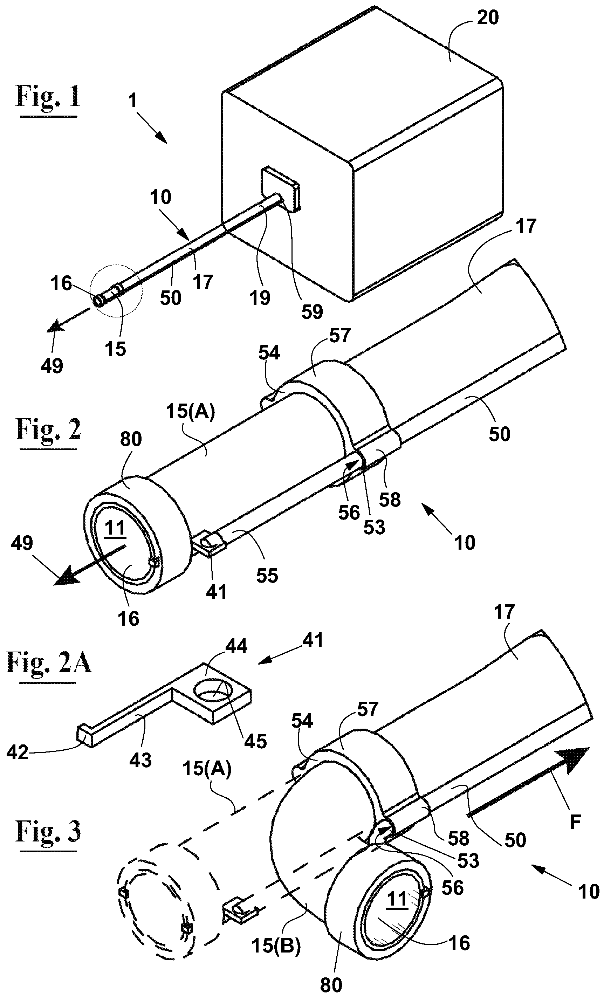

With reference to Figs. 1 - 3, a device 1 is described 1, according to the invention, for endoscopically delivering at least one therapeutic substance. Such device comprises a cannula 10 that defines an extrusion channel 11 and that comprises a proximal portion 19, a flexible distal portion 15 with an extrusion mouth 16 at its own distal end, and a substantially rigid intermediate portion 17 between flexible distal portion 15 and proximal portion 19.

Proximal portion 19 is configured to feed and push a therapeutic substance 49 into extrusion channel 11, in cooperation with an actuation unit 20, so that therapeutic substance 49 can pass through extrusion channel 11 flowing through intermediate portion 17 and through flexible distal portion 15, and can exit from extrusion mouth 16.

Device 1 also comprises at least one actuation cable 50, a proximal end 59 of which can be operated, for example it can be pull, at proximal portion 19 of cannula 10, i.e., of actuation unit 20, as exemplified hereinafter.

Actuation cable 50 also has a distal end 55 connected to flexible distal portion 15 of cannula 10 at extrusion mouth 16, for example, by an anchor 41 shown in detail in Fig. 2A. Anchor 41 comprises a distal fastening portion 42 configured to engage with an edge of extrusion mouth 16, a proximal engagement portion 44 including an engagement means to engage with distal end 55 of actuation cable 50, for example in the form of a hole 45 for engagement with a pin or the like, not shown, connected to distal end 55, and an intermediate portion 43 of connection between distal fastening portion 42 and proximal engagement portion 44.

In this way, or in other possible ways, distal end 55 of actuation cable 50 is configured to displace, in particular to pull flexible distal portion 15 of cannula 10 by a force F transversal to extrusion channel 11, bringing flexible distal portion 15 from an undeformed conformation A, shown in Figs. 2 and, by dashed line, in Fig. 3, to a deformed conformation B shown in Fig. 3.

According to an aspect of the invention, substantially rigid intermediate portion 17 has a distal rigid edge 54 from which flexible distal portion 15 protrudes, and wherein distal rigid edge 54 has a passageway exit 53 of a passageway 56 for actuation cable 50. Distal rigid edge 54 and passageway exit 53 of passageway 56 are arranged in such a way that only flexible distal portion 15 of cannula 10 can be brought from the undeformed conformation A to deformed conformation B by actuation cable 50, while intermediate portion 17 remains undeformed.

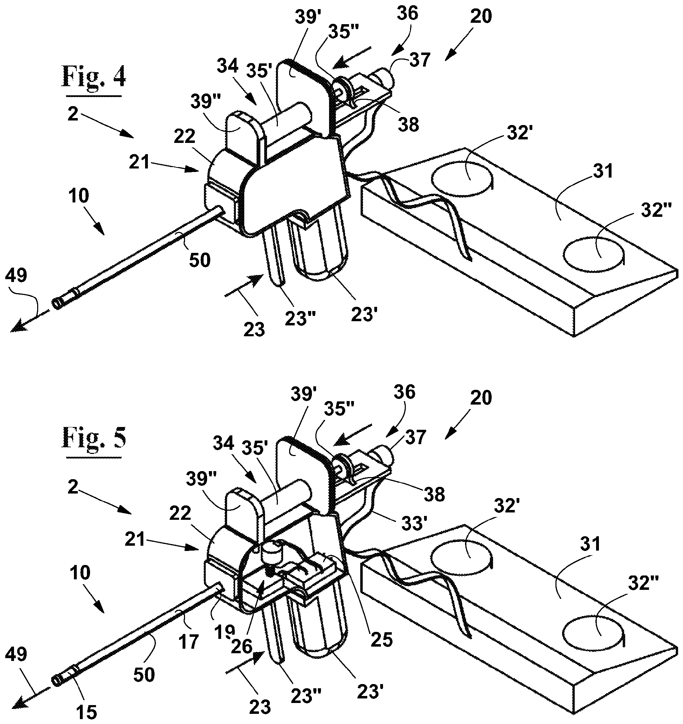

Figs. 4-7 show a device 2 according to a possible exemplary embodiment of actuation unit 20 for causing the deformation of flexible distal portion 15 of cannula 10 and the delivery by extrusion of therapeutic substance 49 through cannula 10 and extrusion mouth 16 thereof. Actuation unit 20 comprises a support 22, in this case in the form of a housing 22, to which motor units 26 and 36 are integrally connected for causing the deformation of flexible distal portion 15 by actuation cable 50, and the delivery of therapeutic substance 49, through cannula 10, respectively. Actuation unit 20 also comprises a control unit 25 functionally connected to motor units 26 and 36.

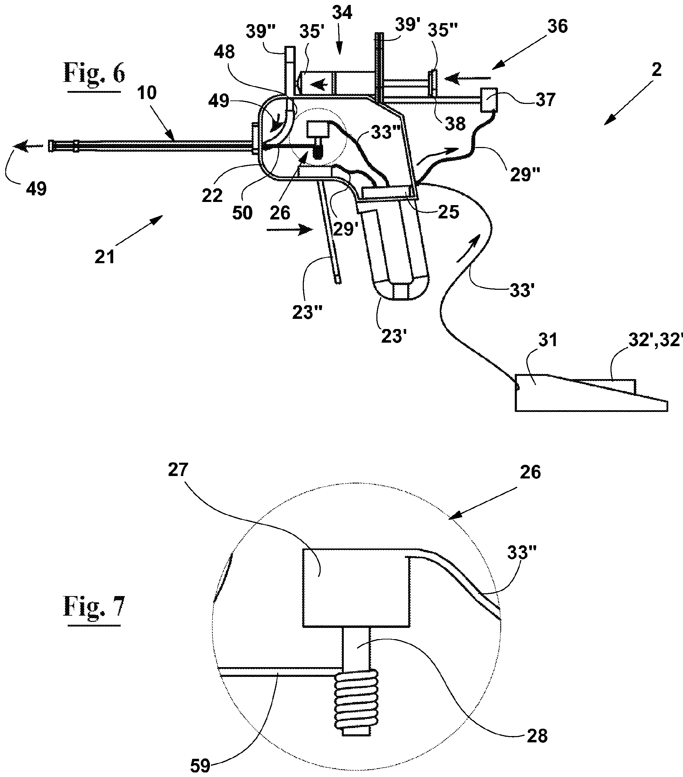

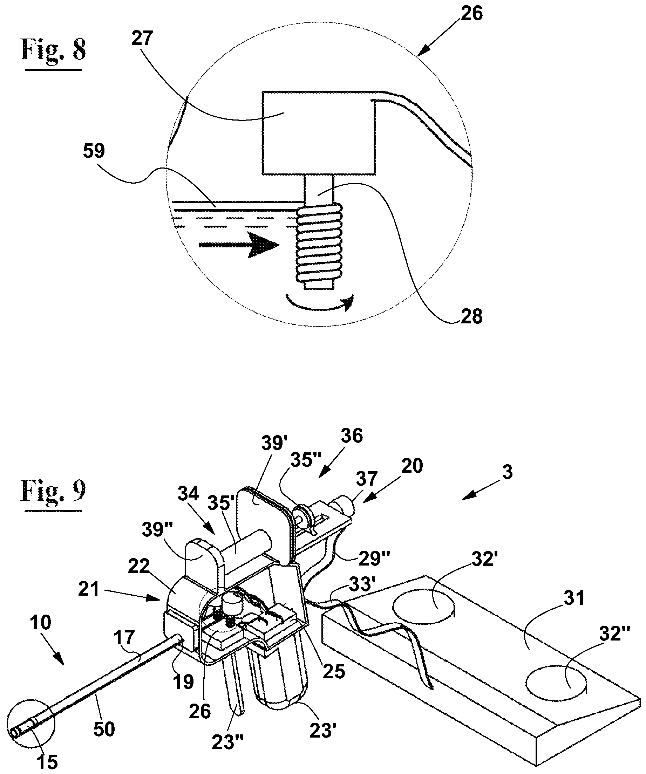

In order to cause the deformation of flexible distal portion 15, motor unit 26 comprises a motor 27 and a shaft 28 that can be brought into rotation by motor 27. Proximal portion 59 is fixed to and wound about shaft 28, as Figs. 7 and 8 show in detail. For this service, actuation unit 20 comprises an interface unit 31 wherein a pushbutton panel includes keys 32' and 32" and is configured to receive a manual deformation input through keys 32',32", and to generate an actuation command signal for actuating the deformation of flexible distal portion 15. A cable 33' is arranged for transferring the actuation command signal to control unit 25, which is in turn connected to motor 27 of motor unit 26 through a cable 33". Upon receiving the deformation command signal, motor 27 causes shaft 28 to rotate, thus increasing the part of proximal portion 59 of actuation cable 50 that is wound about shaft 28, as show still Figs. 7 and 8, and so deforming, flexible distal portion 15 of cannula 10 from the undeformed conformation towards deformed conformation B, until it reaches deformed conformation B or an intermediate conformation between A and B, as required for the delivery by extrusion.

In this case, the therapeutic substance to be extruded is enclosed in a syringe device 34 that comprises a cylinder 35' connected between two brackets 39', 39" protruding from housing 22 and also comprises a piston 35" slidingly arranged in cylinder 35'.

In order to cause the therapeutic substance extrusion, motor unit 36 comprises a motor 37 and a slide 38 connected to motor 37 and integral to piston 35". For this service, actuation unit 20 comprises a gun portion 21 in which a lever 23" is arranged to be shifted by the hand fingers of an operator by grasping the butt 23' of gun portion 21. Gun portion 21 comprises a drive module 24 configured to receive a manual delivery input through the movement of lever 23', as indicated by arrow 23, and to generate a delivery command signal. A cable 29' is arranged for transferring the delivery command signal to control unit 25, which is in turn connected to motor 37 of motor unit 36 through a cable 29". Upon receiving the delivery command signal, motor 37 causes slide 38 and piston 35" to move, thus pushing the therapeutic substance enclosed in cylinder 35' into extrusion channel 11, as shown in Fig. 6, and finally causing the extrusion thereof through extrusion mouth 16.

Figs. 1-8 refer to exemplary embodiments of the invention in which a single actuation cable 50 is provided for deforming flexible distal portion 15 of cannula 10. In this case, advantageously, flexible distal portion 15 is resiliently compliant. This way, by releasing actuation cable 50 after stretching it, flexible distal portion 15 tends to resiliently return from deformed conformation B, or from an intermediate conformation between the configurations B and A, to undeformed conformation A.

In any case, it falls within the scope the invention also the case in which an actuation cable 50 is also configured to push and to pull flexible distal portion 15, in order to bilaterally operate a flexible distal portion 15 of cannula 10 that is not resilient by a single cable 50.

Instead, with reference to Figs. 9-17, a device 3 is described comprising a first actuation cable 50' and a second actuation cable 50" that are arranged to pull flexible distal portion 15 in opposite directions with respect to each other. In this case, actuation cables 50',50" are arranged along diametrically opposite generatrix lines of cannula 10, in particular along diametrically opposite generatrix lines of flexible distal portion 15, or in any case on opposite sides with respect to a longitudinal axis, not shown, of cannula 10.

This exemplary embodiment of the device is preferred, in particular when flexible distal portion 15 is not resiliently compliant, or in any case is not resiliently compliant enough to bring to return to its initial or undeformed conformation A shown in Fig. 9 upon releasing such an actuation cable as actuation cable 50 of Figs. 1-8.

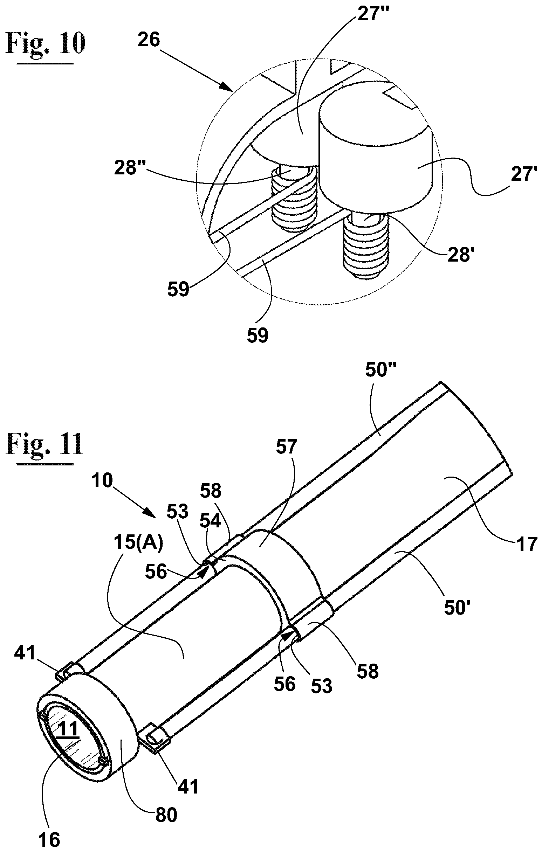

In particular, Figs. 9 and 10 refer to an actuation unit 20 similar to a corresponding actuation unit 20 of device 2 of Figs. 4-8, from which it differs in that motor unit 26 for causing the deformation of flexible distal portion 15 comprises two motors 27', 27" having respective shafts 28', 28" for operating tie-member 50' and tie-member 50", respectively. Even in this case, the proximal portions of tie members 50',50" are fixed and partially wound about shafts 28', 28", respectively.

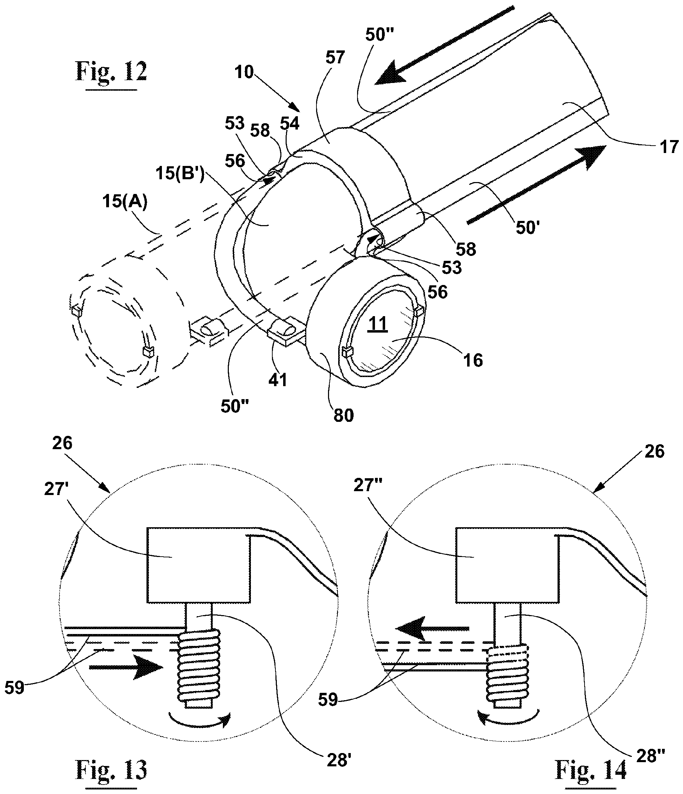

Fig. 12 shows flexible distal portion 15 of cannula 10 in a first deformed conformation B'. In order to attain first deformed conformation B', motor 27' is operated, so as to cause shaft 28' to rotate in such a rotation direction that tie-member 50' is wound thereon, as shown in Fig. 13, while a part of the proximal portion of tie-member 50" wound about shaft 28" connected to motor 27" is unwound from shaft 28", as shown in Fig. 14, so as to allow the deformation of flexible distal portion 15.

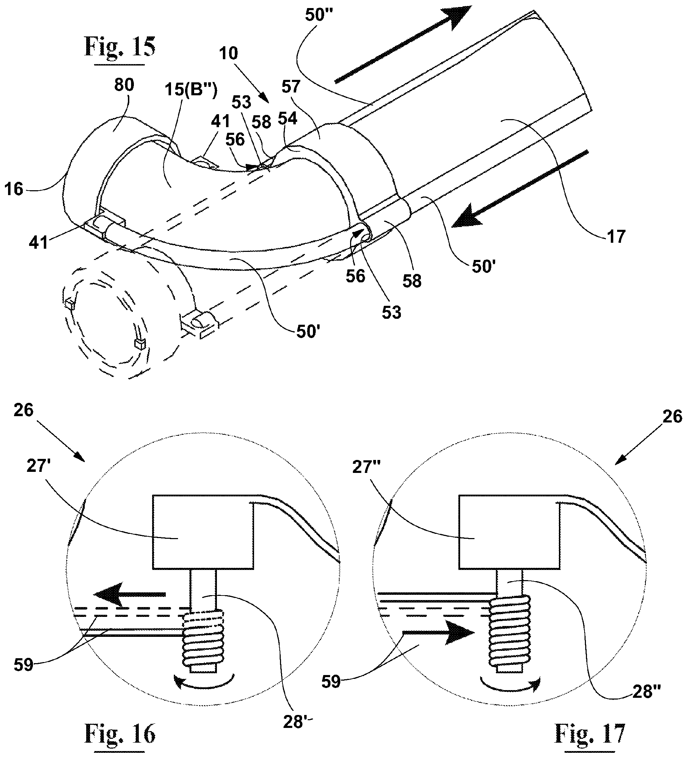

Similarly, Fig. 15 shows flexible distal portion 15 of cannula 10 in a second deformed conformation B". To attain second deformed conformation B", motor 27" is operated, so as to cause shaft 28" to rotate in such a rotation direction that tie-member 50" is wound thereon, as shown in Fig. 17, while a part of the proximal portion of tie-member 50' wound about shaft 28' connected to motor 27' is unwound from shaft 28', as shown in Fig. 16, so as to allow a deformation of flexible distal portion 15.

In other words, first actuation cable 50' and second actuation cable 50" can selectively work as an agonist actuation cable and an as antagonist actuation cable, respectively, and vice-versa, in the reverse order, as an antagonist actuation cable and as an agonist actuation cable, so as to deform flexible distal portion 15 in an opposite direction with respect to a longitudinal axis of cannula 10, thus obtaining two opposite deformed conformations B', B" of flexible distal portion 15 and two deformed orientations, i.e., two orientation angularly spaced apart by 180°, of extrusion mouth 16 of cannula 10.

Both such deformations of flexible distal portion 15 of cannula 10 of device 3 take place in a deformation plane, not shown, defined by the longitudinal axis of cannula 10 and by actuation cables 50',50" diametrically opposite to each other, similarly to the deformation of flexible distal portion 15 of cannula 10 of devices 1 and 2, which takes place in a deformation plane, not shown, defined by the longitudinal axis of cannula 10 and by actuation cable 50 (Fig. 3). The extrusion mouth is then oriented, and its own middle axis covers an angle, for example a 180° angle, lying in this deformation plane.

The operator can obtain lateral orientations of extrusion mouth 16 other than the orientations corresponding to deformed conformations B,B',B" of Figs. 3, 13, 15 by rotating the deformation plane, i.e., by rotating cannula 10 about its own longitudinal axis, within a 360° angle in the case of devices 1 and 2 having a single actuation cable 50, and within a 180° angle in the case of device 3 having two actuation cables 50.

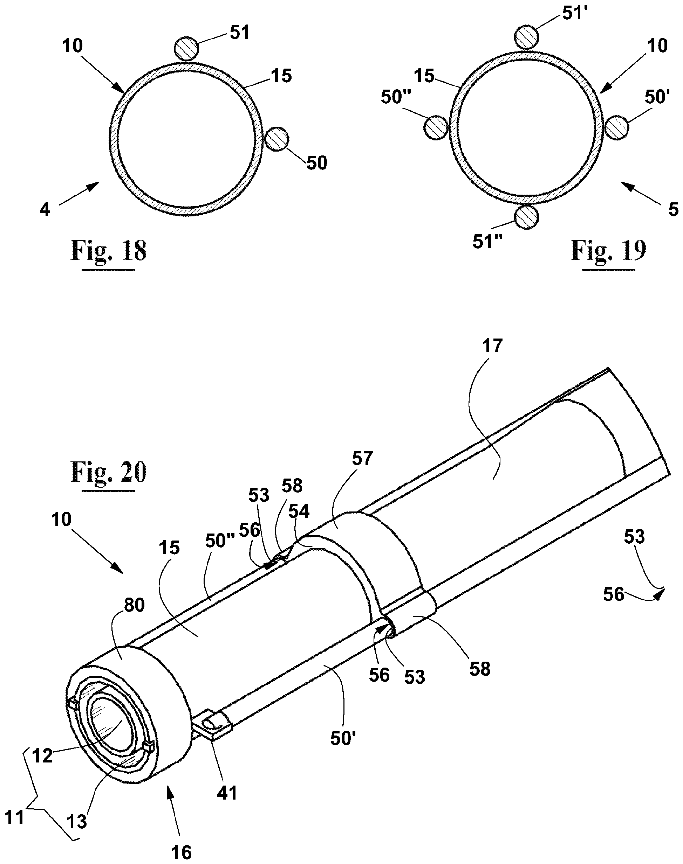

As an alternative, in order to obtain a generic lateral orientation of delivery mouth 16 about the axis of cannula 10 by a minimum rotation or by no rotation of cannula 10 about its own longitudinal axis, devices 4 and 5 can be used, as shown in the respective cross-sectional views of Figs. 18 and 19, made by sectional planes transversally arranged to cannula 10, for example, to flexible distal portion 15 of cannula 10.

In device 4 of Fig. 18, besides actuation cable 50, at least one further actuation cable 51 is provided, for example a further actuation cable angularly distant by 90° from actuation cable 50 about longitudinal axis of cannula 10. Further actuation cable 51 has a distal end, not shown, connected to flexible distal portion 15 at extrusion mouth 16, and configured to pull flexible distal portion 15 and bring it from undeformed conformation A to a further deformed conformation that lies in a plane different from the plane where deformed conformation B lies, which is defined by actuation cable 50 and by the longitudinal axis of cannula 10.

By device 4, the orientations of delivery mouth 16 out of the plane can be obtained by suitably combining the pulling forces acting on actuation cables 50 and 51. Similarly to devices 1 and 2, device 4 is adapted to provide an unilateral actuation that is well suited if flexible distal portion 15 is resiliently compliant enough to spontaneously return to undeformed conformation A upon releasing actuation cables 50, 51.

In device 5 of Fig. 19, besides actuation cables 50',50" of device 3 of Figs. 9-17, at least two further actuation cables 51',51" are provided, for example further actuation cables that are rotationally spaced apart by 90° from actuation cables 50',50", respectively, about the longitudinal axis of cannula 10. Further actuation cables 51',51" have respective distal ends, not shown, connected to flexible distal portion 15 at extrusion mouth 16, and configured to pull flexible distal portion 15 and to bring it from undeformed conformation A to further deformed conformations that are diametrically opposite to each other and that can be arranged in a plane different from the deformation plane defined by actuation cables 50',50" and by the longitudinal axis of cannula 10.

By device 5, the orientations of delivery mouth 16 out of the plane can be obtained by suitably combining the pulling forces acting on cables 50',50" and 51',51". Similarly to device 3, device 5 is particularly suitable if the flexible distal portion is not resiliently compliant enough to spontaneously return to its own undeformed conformation A upon releasing actuation cables 50',50", 51',51".

As Figs. 2, 3, 11, 12, 15 show, the device preferably comprises at least one guide element 58 for each actuation cable 50,50',50". This guide element 58 has preferably the shape of a cylinder and is arranged along an outer surface of intermediate portion 57 of cannula 10;

Fig. 20 is a partial view of a cannula 10 according to an exemplary embodiment in which extrusion channel 11 of an extrusion device is configured as a central extrusion channel 12 and an annular extrusion channel 13 coaxial to each other. With no purpose of limitation, this extrusion device relates to an exemplary embodiment of Figs. 9-17, in which are provided two actuation cables 50',50". Proximal portion 19 of cannula 10, in cooperation with actuation unit 20, is configured to feed and push a first therapeutic substance into central extrusion channel 12 and a second therapeutic substance into annular extrusion channel 13, and to deliver the therapeutic substances through extrusion mouth 16.

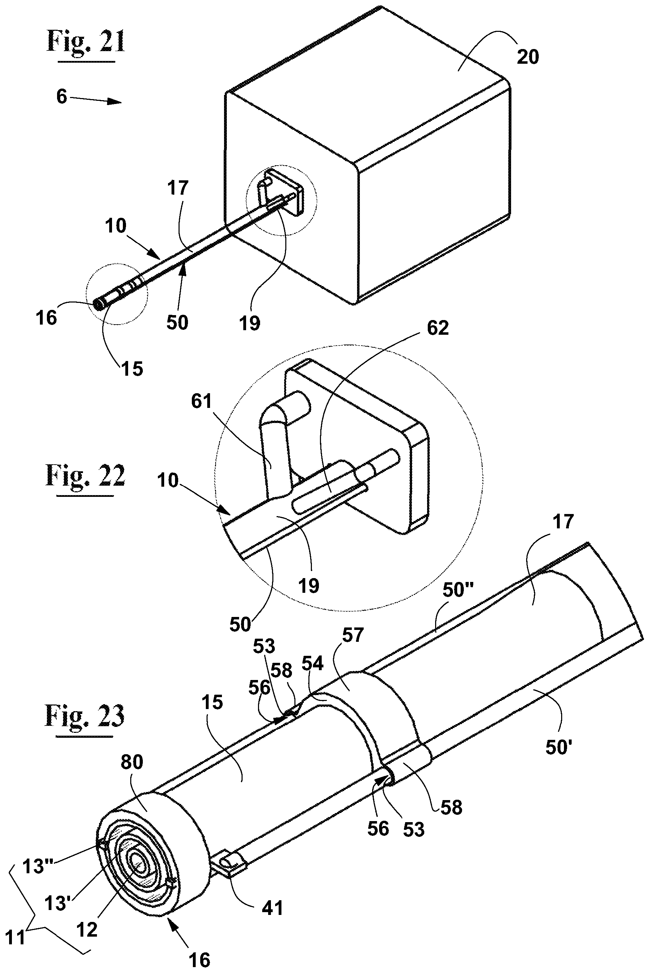

With reference to Figs. 21-26, a device 6 is described according to an exemplary embodiment of the invention, in which extrusion channel 11 is configured as a central extrusion channel 12, a first annular extrusion channel 13' and a second annular extrusion channel 13" coaxial to one another. Proximal portion 19 of cannula 10, in cooperation with actuation unit 20, is configured to feed and push a first therapeutic substance into first annular extrusion channel 13' and a second therapeutic substance into second annular extrusion channel 13", or a first therapeutic substance into central extrusion channel 12, a second therapeutic substance into first annular extrusion channel 13' and a third therapeutic substance into second annular extrusion channel 13", and to deliver the therapeutic substances through extrusion mouth 16.

More in detail, three connection ducts 61, 62, only two of which shown in Fig. 22, are arranged at proximal portion 19 of cannula 90 for conveying the therapeutic substances from respective reservoirs, for example from respective syringe devices as syringe device 34 of Figs. 4-6, to extrusion channels 12, 13',13" through a convergence chamber towards the axis of cannula 10 arranged upstream of concentric extrusion channels 12, 13',13".

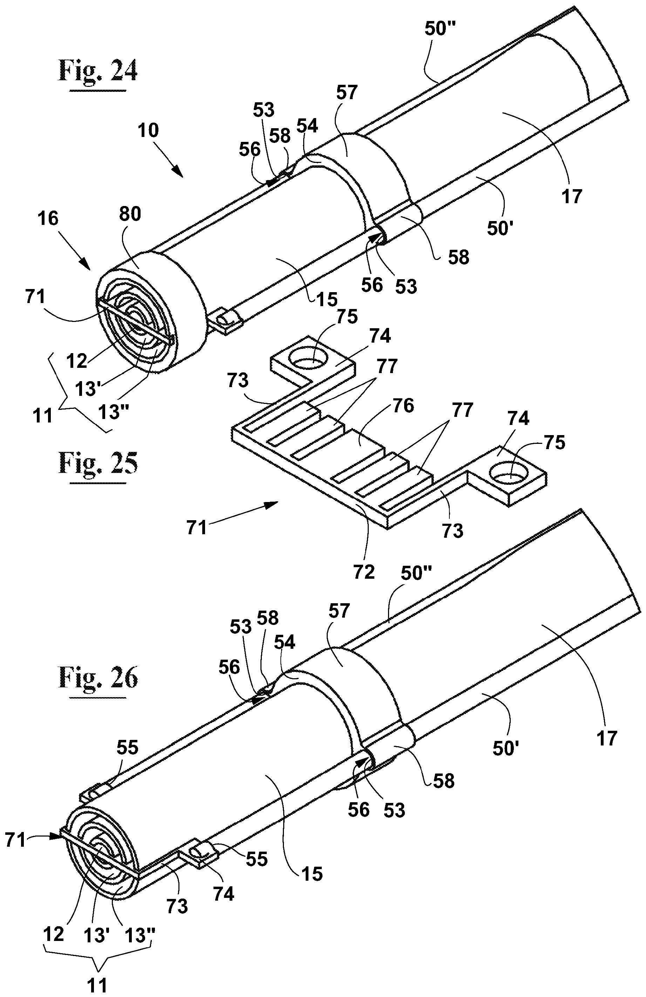

In an advantageous modification of device 6, as shown in Figs. 24-26, at extrusion mouth 16, a substantially comb-shaped rigid spacer element 71 is provided, comprising a support rod 72 and of a plurality of teeth 76,77 protruding from a same side of support rod 72. More in detail, the teeth comprise a central tooth 76 to be diametrically introduced into central extrusion channel 12 and four teeth 77 to be two by two introduced into first and second annular extrusion channels 13' and 13", in order to keep the separation walls of extrusion channels 12, 13',13" and the external wall of flexible distal portion 15 spaced apart from one another, so as to maintain extrusion mouth 16 open when flexible distal portion 15 moves from undeformed conformation A to deformed conformation B,B'. In an advantageous modification, as shown in Fig. 25, spacer element 71 also comprises a pair of engagement portions 74 to engage with the distal portions of actuation cables 50',50", as well as two respective intermediate portions 73 connecting support rod 72 with engagement portions 74. Distal end 55 of each actuation cable 50',50" is connected to a respective engagement portion 74.

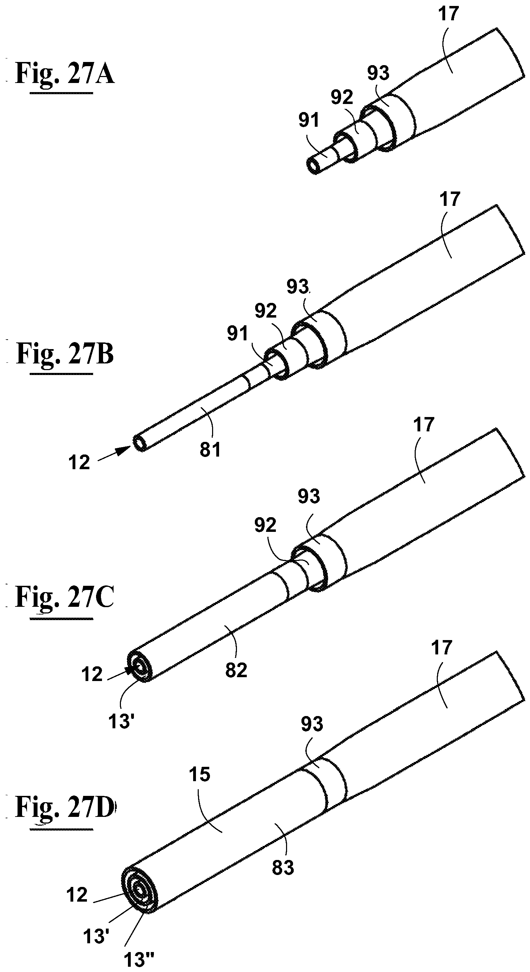

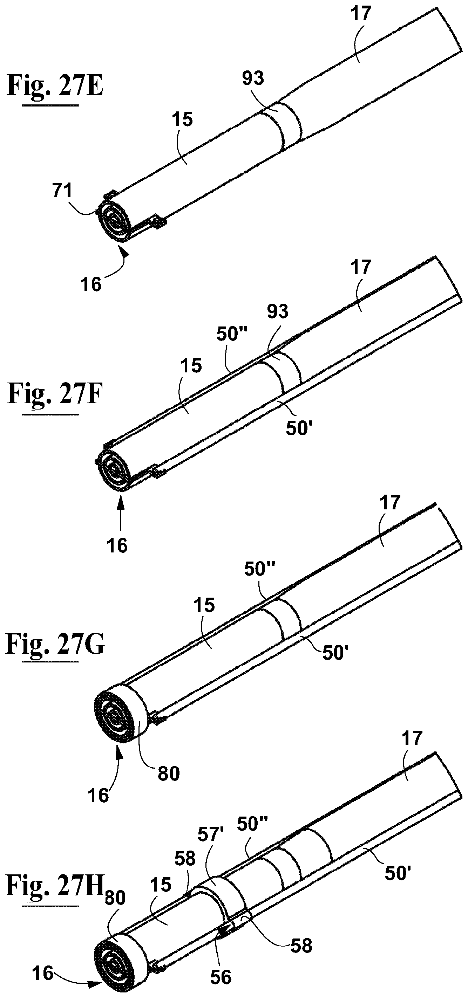

Figs. 27A-27H show a manufacturing cycle of flexible distal portion 15 of a cannula 10 by insertion of concentric cylindrical walls 81, 82, 83. More in detail, as shown in Fig. 27A, rigid intermediate portion 17 comprises three fastening portions 91, 92, 93 for respective concentric cylindrical walls 81, 82, 83 defining extrusion channels 12, 13' and 13". Figs. 27B, 27C and 27D show the result of the insertion of flexible cylindrical walls 81, 82, 83. Moreover, Fig. 27E shows rigid spacer element 71 of Fig. 25 with teeth 76,77 inserted in extrusion channels 12, 13',13". Moreover, Fig. 27F shows actuation cables 50',50" connected to engagement portions 74 of rigid spacer element 71. Moreover, Fig. 27G shows a collar 80 inserted on flexible distal portion 15 at extrusion mouth 16. Moreover, Fig. 27H shows a guide element 58 for each actuation cable 50',50".

As an alternatively to what is shown in Figs. 27A-27H, flexible distal portion 15 of cannula 10 can be manufactured in a heat-shrinking material, so that it can be fixed to intermediate portion 17 by an insertion step of intermediate portion 17 into the distal portion and by a subsequent thermal cycle configured to form a force-fitting connection between flexible distal portion 15 and rigid intermediate portion 17.

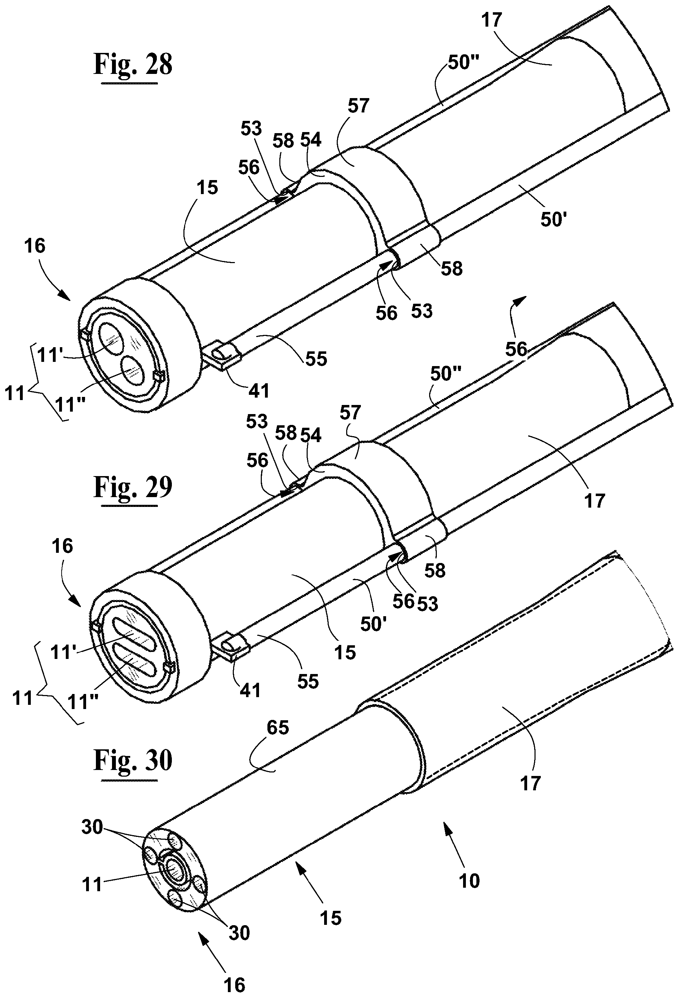

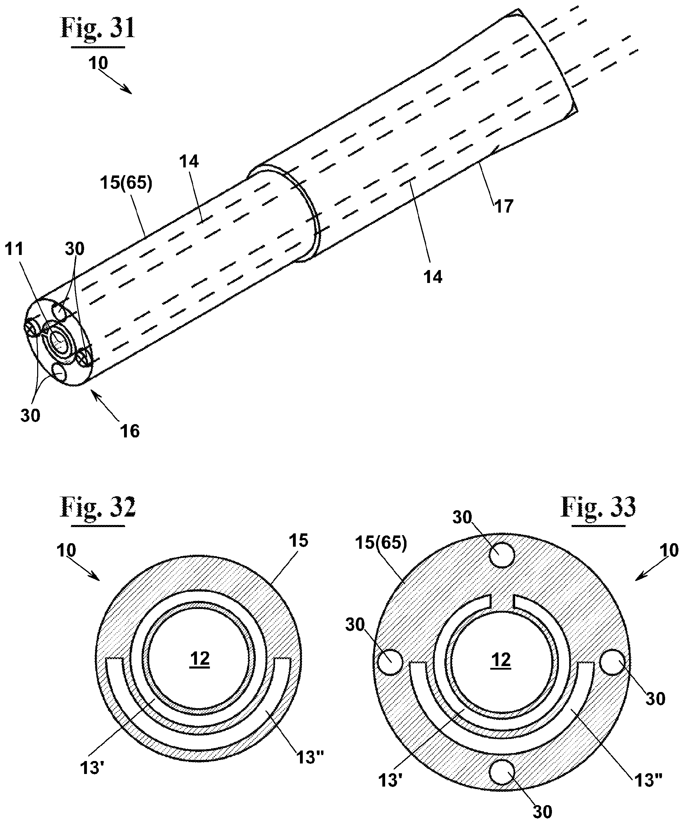

As Figs. 30 and 31 show, according to another aspect of the invention, flexible distal portion 15 of cannula 10 can be manufactured from a multi-lumen tube 65 made of a flexible material and having a plurality of longitudinal channels, by at least partially arranging this multi-lumen tube 65 into a part of intermediate portion 17 of cannula 10, so that a portion of the former protrudes out of the latter by predetermined length, said protruding portion forming flexible distal portion 15.

Still according to this aspect of the invention, also passageways 30 for respective actuation cables are provided by further lumina of multi-lumen tube 65 as shown, for example, in Figs. 30, 31 and 33.

Figs. 28 and 29, instead, relate to devices according to different modifications of an exemplary embodiment of the invention, in which extrusion channel 11 comprises a first extrusion channel 11' and a second extrusion channel 11" parallel to each other, and proximal portion 19, in cooperation with actuation unit 20, for example as shown in Fig. 4, is configured to feed and push a first and a second therapeutic substance into the first and into the second extrusion channel 11',11", respectively, and to cause such therapeutic substances to be delivered through extrusion mouth 16 when the flexible distal portion 15 is in deformed conformation B.

More in detail, in the embodiment modification of Fig. 28 first and second extrusion channels 11',11" have a circular cross section. Instead, in the embodiment modification of Fig. 29, first and second extrusion channels 11',11" have a substantially rectangular cross section, with a ratio higher than 10 between adjacent sides, preferably higher than 20, to obtain a laminar flow of the therapeutic substance being extruded through extrusion mouth 16.

Flexible distal portion 15 has a length L‴ set between 1 mm and 10 mm, and is configured so that deformed conformation B has the shape of an arc.

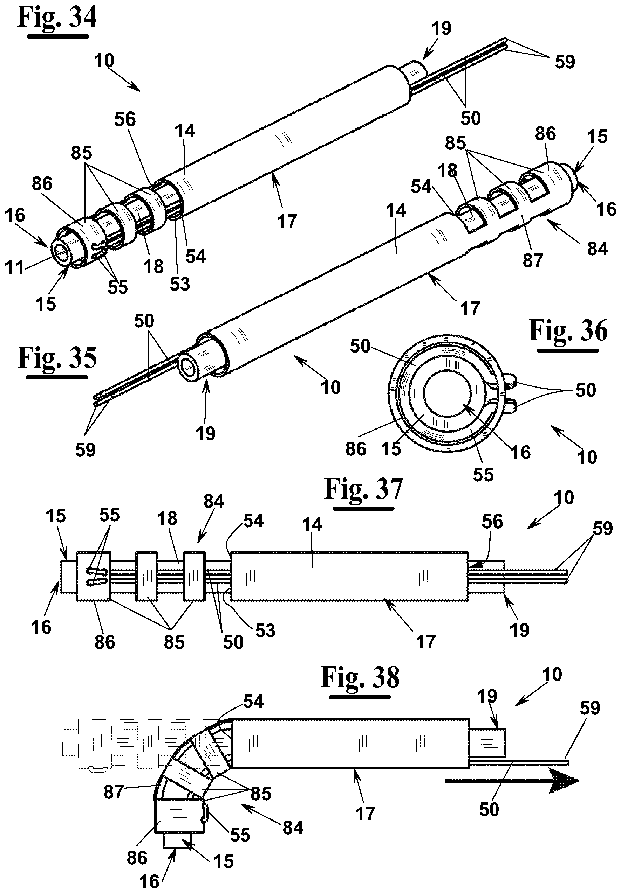

With reference to Figs. 34-38, a cannula 10 is described of a device according to an advantageous exemplary embodiment of the invention, in which a flexible rod 84 extends from distal rigid edge 54 of substantially rigid intermediate portion 17, said flexible rod comprising a plurality of ring portions 85,86 that surround flexible distal portion 15 of cannula 10, and within which an actuation cable 50 is slidingly arranged, in particular, within an annular end portion 86 to which distal end 55 of actuation cable 50 is connected. This way, when pulling actuation cable 50, flexible rod 84 bends and ring portions 85,86 follow and guide flexible distal portion 15 of cannula 10 from undeformed conformation A (Figs. 34-37) to deformed conformation B (Fig. 38). In a shown modification of the present exemplary embodiment, shown in the figures, actuation cable 50 can comprise two branches parallel to each other, and distal end 55 has the shape of a connection loop for connection to annular end portion 86 (Fig. 36).

In particular, in a further advantageous modification of the present exemplary embodiment, distal portion 15 and intermediate portion 17 of cannula 10 comprise a common tubular core element 18 which is made of a flexible material, typically a polymeric material, and that defines extrusion channel 11, and also comprise a shell element 14 enclosing tubular core element 18, which is also a common element of distal and intermediate portions 15, 17 of cannula 10. Shell element 14 is preferably made of a inherently rigid material, or in any case in a material more rigid than the material of tubular core element 18, in particular it can be made of a metal material such as a nickel-titanium alloy, for instance, the Nitinol alloy. Moreover, shell element 14 comprises distal rigid edge 54 and flexible rod 84.

More in particular, flexible rod 84 comprises an elongated support portion 87 extending parallel to the longitudinal axis of cannula 10 starting from a sector of distal edge 54, and ring portions 85,86 extend between two opposite lateral sides 88, 89 of elongated support portion 87, transversally to elongated support portion 87.

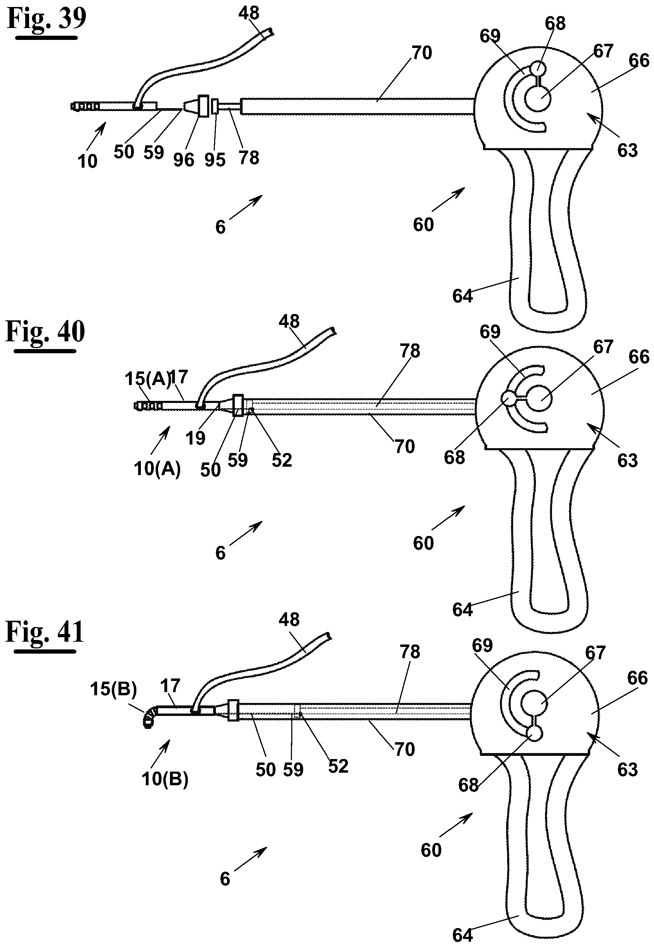

Figs. 39-43 show devices 6, 7 and 8 according to further exemplary embodiments of actuation unit 60, 60' and 60" to perform the deformation of flexible distal portion 15 of cannula 10. In these cases, actuation unit is configured as a purely mechanical, manually operated actuation unit, as an alternative to driven actuation unit 20 of device 2 (Figs. 4-7). Advantageous embodiment modifications of devices 6, 7 and 8 (Figs. 39, 42, 43) comprise a cannula 10 arranged for being removed from the device and replaced such as the only disposable part of devices 6, 7 and 8.

In devices 6, 7 and 8, manual actuation unit 60 is combined with a cannula 10, as shown in Figs. 34-38, however it is adapted to operate cannulas according to different embodiments of the invention, for instance, cannula 10 shown in Figs. 1-3, as well as driven actuation unit 20 of Figs. 4-7 is adapted to operate cannula 10 of Figs. 34-38.

Actuation unit 60 comprises a handle portion 64 and a housing 66 integral to each other. Housing 66 includes a manual actuation mechanism 63, 63', 63" connected to proximal end 59 of actuation cable 50, which are arranged for pull actuation cable 50 in order to position cannula 10 in device 6, 7 and 8, as described hereinafter, and to bend the end distal portion of cannula 10 from the undeformed configuration to of Fig. 40 towards / up to deformed configuration B of Fig. 41, in the case of device 6, and similar configurations, in the case of devices 7 and 8.

Manual actuation mechanism 63, 63', 63" comprises an actuation axis 67, and an actuation rod 78 that is slidingly arranged in a linear extension 70 of housing 66 and integrally connected to actuation axis 67 in a way not shown but obvious for a skilled person. Proximal end 59 of actuation cable 50 is connected to distal end 95 of actuation rod 78, for example, by a knot or by another releasable connection.

Actuation axis 67 of device 6 of Figs. 39-41 is rotatably arranged within housing 66. A knob 68 extends radially from actuation axis 67 and is arranged to cause actuation axis 67 to rotate and therefore actuation rod 78 to translate, in order to pull/release actuation cable 50 of cannula 10. Housing 66 has an annulus-shaped opening 69, in this case a 180° annulus sector, through which knob 68 protrudes, so that to be manipulated by an operator who grasps handle portion 64.

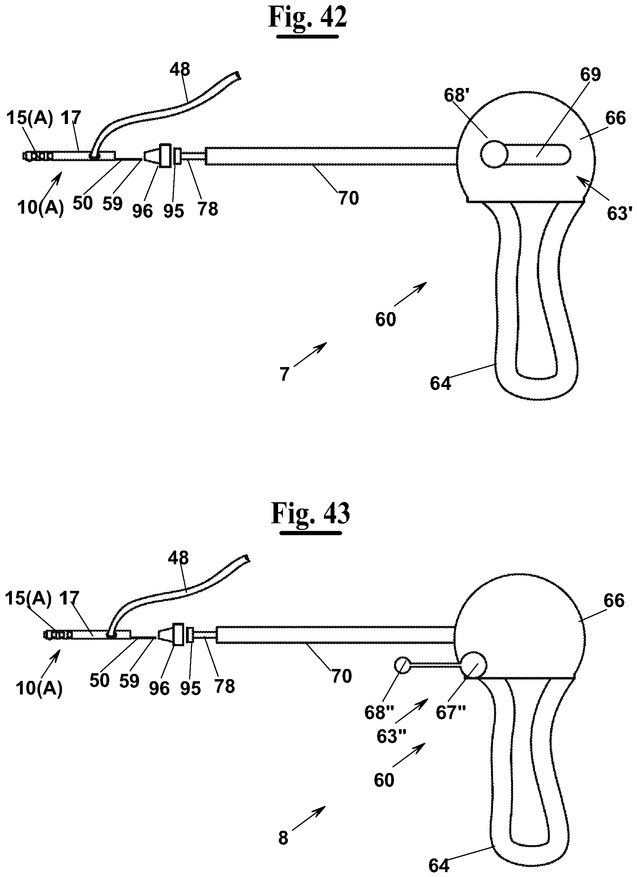

Actuation mechanism 63' of device 7 of Fig. 42 differs from actuation mechanism 63 of device 6 in that it comprises a slidable actuation axis 67' with an end knob 68' that protrudes out of a linear opening 69 of housing 66 and is arranged to cause actuation axis 67 to translate and actuation rod 78 to translate as well, in order to pull/release actuation cable 50 of cannula 10.

Actuation mechanism 63" of device 8 of Fig. 43 differs from actuation mechanism 63 of device 6 in that rotatable actuation axis 67" is arranged in a peripheral position of housing 66, and in that knob 68" extends peripherally beyond the outline of housing 66 instead of extending centrally from a groove thereof.

Figs. 39, 42 and 43 is an exploded view of devices 6, 7 and 8, i.e., the devices are shown in a removed configuration of cannula 10. For bringing such devices by the removed configuration to the undeformed configuration of Fig. 40, proximal end 59 is connected to proximal end 95 of actuation rod 78 as described above, by introducing proximal end 19 into a recess of a connection fitting 96 connected to distal end 95 of actuation rod 78 and by tightening actuation cable 50 by knob 68, 68', 68" and by actuation axis 67, 67', 67" as it can understood from the above description, causing knob 68, 68', 68" to perform a first stroke. In case of device 6, the first stroke extends by a portion of the angle defining annulus-shaped opening 69, for example by the half of the angle, in the shown case this angle portion is 90°.

In order to bring distal portion 15 of cannula 10 from the undeformed configuration A of Fig. 40 to the deformed configuration B of Fig. 41, or to an intermediate configuration between the configurations A and B, the knob is caused to perform a second stroke towards a limit position of knob 68, in this case a maximum extension of the second stroke corresponds to a remaining portion of the angle defining annulus-shaped opening 69. Similar actions on knobs 68' and 68" of devices 7 and 8 can be easily deducted from the figures. For the sake of simplicity, devices 7, 8 are shown in Figs. 42 and 43, respectively, with distal portion 15 of cannula 10 only in the removed configuration, since undeformed configuration A and deformed configuration B of distal portion 15 appear to be obvious.

The foregoing description exemplary embodiments and embodiment modifications of the invention will so fully reveal the invention according to the conceptual point of view, so that others, by applying current knowledge, will be able to modify and/or adapt for various applications such embodiment without further research and without parting from the invention, and, accordingly, it is meant that such adaptations and modifications will have to be considered as equivalent to the exemplary embodiments and exemplary specific embodiments. The means and the materials to realise the different functions described herein could have a different nature without, for this reason, departing from the field of the invention. It is to be understood that the phraseology or terminology that is employed herein is for the purpose of description and not of limitation.

The invention is defined by the following claims.

## Claims

1. A device for endoscopically delivering at least one therapeutic substance into a small articular cavity, said device comprising:
- a cannula (10) defining at least one extrusion channel (11) and comprising:
- a proximal portion (19);
- a flexible distal portion (15) having an extrusion mouth (16) and
- a substantially rigid intermediate portion (17) between said flexible distal portion (15) and said proximal portion (19),
wherein said proximal portion (19) is configured to feed and push a therapeutic substance into said extrusion channel (11), so that said therapeutic substance can pass through said extrusion channel (11) flowing through said intermediate portion (17) and through said flexible distal portion (15), and can then exit from said extrusion mouth (16),
further comprising:
- at least one actuation cable (50,50',50") having:
- a proximal end (59) operable at said proximal portion (19) of said cannula (10);
- a distal end (55) connected to said flexible distal portion (15) of said cannula (10) at said extrusion mouth (16), and configured to pull said flexible distal portion (15) with a force transversal to said extrusion channel (11), bringing said flexible distal portion (15) from an undeformed conformation (A) to a deformed conformation (B,B',B")
**characterized in that**
said substantially rigid intermediate portion (17,57) has a distal rigid edge (54) from which said flexible distal portion (15) protrudes, and wherein said distal rigid edge (54) has a passageway exit (53) of a passageway (56) for said actuation cable (50,50',50"), said distal rigid edge (54) and said passageway exit (53) of said passageway arranged in such a way that only said flexible distal portion (15) of said cannula (10) can be brought, by said actuation cable (50,50',50"), from said undeformed conformation (A) to said deformed conformation (B,B',B"), in order to reach said small articular cavity, while said intermediate portion (17) is always undeformed.

2. The device according to claim 1, wherein a flexible rod (84) extends from said distal rigid edge (54), said flexible rod (84) comprising a plurality of ring portions (85,86) that surround said flexible distal portion (15) of said cannula (10), wherein said plurality of ring portions (85,86) comprises a terminal ring (86) to which said distal end (55) of said actuation cable (50) is connected, in such a way that, by pulling said actuation cable (50), said flexible rod (84) bends and said ring portions (85,86) follow and guide said flexible distal portion (15) of said cannula (10) from said undeformed conformation (A) to said deformed conformation (B), said actuation cable (50) arranged to slide in said ring portions (85,86).

3. The device according to claim 2, wherein said distal portion (15) and said intermediate portion (17) of said cannula (10) comprise:
- a common tubular core element (18) made of a flexible material, said tubular core element (18) defining said extrusion channel (11);
- a common shell element (14) enclosing said tubular core element (18),
wherein said shell element (14) comprises said distal rigid edge (54) and said flexible rod (84).

4. The device according to claim 3, wherein said flexible rod (84) comprises an elongated support portion (87) extending parallel to a longitudinal axis of said cannula (10) from a sector of said distal edge (54), said ring portions (85,86) extending transversally to said elongated support portion (87) between two opposite lateral sides (88, 89) of said elongated support portion (87).

5. The device according to claim 1, wherein said flexible distal portion (15) of said cannula (10) is resiliently compliant, whereby, by releasing said actuation cable (50) after stretching it, said flexible distal portion (15) returns from said deformed conformation (B) to said undeformed conformation (A).

6. The device according to claim 1, wherein said at least one actuation cable is a first actuation cable (50'), and a second actuation cable (50") is provided configured to pull said flexible distal portion (15) in an opposite direction with respect to said first actuation cable (50'), so that said first actuation cable (50') and said second actuation cable (50") can selectively work as an agonist actuation cable (50',50") and an antagonist actuation cable (50",50'), and vice-versa.

7. The device according to claim 1, wherein at least one further actuation cable (51',51") is provided comprising a distal end connected to said flexible distal portion (15) at said extrusion mouth (16), and configured to pull said flexible distal portion (15) bringing it from said undeformed conformation (A) to a further deformed conformation in a plane that is distinct from a plane of said deformed conformation (B,B',B") and is defined by an axis of said cannula (10), and by an axis of said actuation cable (50).

8. The device according to claim 1, wherein said extrusion channel (11) is configured in a way selected from the group comprised of:
- a central extrusion channel (12) and an annular extrusion channel (13) coaxial to each other, and said proximal portion (19) is configured to feed and push a first therapeutic substance into said central extrusion channel (12) and a second therapeutic substance into said annular extrusion channel (13), and to deliver said therapeutic substances through said extrusion mouth (16) with said flexible distal portion (15) in said deformed conformation (B,B',B");
- a central extrusion channel (12), a first annular extrusion channel (13') and a second annular extrusion channel (13") coaxial to one another, and said proximal portion (19) of said cannula (10) is configured to feed and push a first therapeutic substance into said first annular extrusion channel (13') and a second therapeutic substance into said second annular extrusion channel (13"), or a first therapeutic substance into said central extrusion channel (12), a second therapeutic substance into said first annular extrusion channel (13') and a third therapeutic substance into said second annular extrusion channel (13") and to deliver said therapeutic substances through said extrusion mouth (16) with said flexible distal portion (15) in said deformed conformation (B,B',B");
- a first and a second extrusion channel (11',11") parallel to each other, and said proximal portion (19) is configured to feed and push a first and a second therapeutic substance into said first and second extrusion channel (11',11"), respectively, of said parallel extrusion channels and to deliver said therapeutic substances through said extrusion mouth (16) with said flexible distal portion (15) in said deformed conformation (B,B',B").

9. The device according to claim 8, wherein, at said extrusion mouth (16), a rigid spacer element (71) is provided, to which said distal end (55) of said actuation cable (50) is fixed, and wherein said rigid spacer element (71) is configured to engage with and keep open said extrusion mouth (16) when said flexible distal portion (15) moves from said undeformed conformation (A) to said deformed conformation (B,B',B").

10. The device according to claim 9, wherein said rigid spacer element (71) has a substantially comb-like shape, and comprises a support rod (72) and of a plurality of teeth (76,77) protruding from a same side of said support rod (72) and inserted in said central extrusion channel (12) and in said annular extrusion channel (13) or in said first and second annular extrusion channels (13',13"), so as to maintain a predetermined distance between separation walls of said extrusion channels (12,13,13',13"), and an external wall of said flexible distal portion (15), maintaining said extrusion mouth (16) open when said flexible distal portion (15) moves from said undeformed conformation (A) to said deformed conformation (B,B').

11. The device according to claim 9, wherein said spacer element (71) also comprises a pair of engagement portions (74) each engaging with said distal end (55) of a respective actuation cable (50',50"), as well as two respective intermediate portions (73) of connecting said support rod (72) with said engagement portions (74).

12. The device according to claim 1, wherein a wall of said extrusion channel (11), at said flexible distal portion (15), is made as a composite material wherein a compression spring is incorporated in a polymeric tubular matrix, in particular by a dip coating technique.

13. The device according to claim 1, wherein said flexible distal portion (15) of said cannula (10) is made of a heat-shrinking material, so as to anchor said flexible distal portion (15) to said intermediate portion (17) by introducing said intermediate portion (17) into said distal portion and by performing a subsequent predetermined thermal cycle.

14. The device according to claim 1, wherein said flexible distal portion (15) has a length (L‴) set between 1 mm and 10 mm, and is configured so that said deformed conformation (B,B',B") has the shape of an arc.

15. The device according to claim 1, wherein said flexible distal portion (15) is made of a material selected from the group consisting of:
- silicone rubber
- thermoplastic polymers, in particular polycarbonates, polysulfones, polyolefins, in particular polystyrene, polyethylene or polypropylene, polyurethanes, PVC, polyesters, in particular polylactic acid, polyglycolic acid (PGA), polyethylene terephthalate (PET), polyacrylates, in particular polymethylmethacrylate, nylon;
- synthetic elastomers, in particular styrene-butadiene and styrene-butadiene-styrene and polyester rubbers;
- natural rubber, in particular latex (and derivatives thereof).

## Patentansprüche

1. Vorrichtung zur endoskopischen Abgabe von zumindest einer therapeutischen Substanz in eine kleine Gelenkhöhle, wobei die Vorrichtung Folgendes umfasst:
- eine Kanüle (10), die zumindest einen Extrusionskanal (11) definiert und Folgendes umfasst:
- einen proximalen Abschnitt (19); und
- einen flexiblen distalen Abschnitt (15) mit einer Extrusionsmündung (16), und
- einen im Wesentlichen starren Zwischenabschnitt (17) zwischen dem flexiblen distalen Abschnitt (15) und dem proximalen Abschnitt (19), wobei der proximale Abschnitt (19) konfiguriert ist, um eine therapeutische Substanz in den Extrusionskanal (11) zuzuführen und zu drücken, sodass die therapeutische Substanz durch den Extrusionskanal (11) laufen kann, durch den Zwischenabschnitt (17) und durch den flexiblen distalen Abschnitt (15) fließt und dann aus der Extrusionsmündung (16) austreten kann, ferner umfassend:
- zumindest ein Betätigungskabel (50, 50', 50") mit:
- einem proximalen Ende (59), das an dem proximalen Abschnitt (19) der Kanüle (10) bedienbar ist;
- einem distalen Ende (55), das mit dem flexiblen distalen Abschnitt (15) der Kanüle (10) an der Extrusionsmündung (16) verbunden und konfiguriert ist, um den flexiblen distalen Abschnitt (15) mit einer Kraft quer zu dem Extrusionskanal (11) zu ziehen und den flexiblen distalen Abschnitt (15) aus einer unverformten Konformation (A) in eine verformte Konformation (B, B', B'') zu bringen
**dadurch gekennzeichnet, dass**
der im Wesentlichen starre Zwischenabschnitt (17, 57) eine distale starre Kante (54) aufweist, von welcher der flexible distale Abschnitt (15) vorsteht, und wobei die distale starre Kante (54) einen Durchgangsausgang (53) eines Durchgangs (56) für das Betätigungskabel (50, 50', 50") aufweist, wobei die distale starre Kante (54) und der Durchgangsausgang (53) des Durchgangs auf eine solche Weise angeordnet sind, dass nur der flexible distale Abschnitt (15) der Kanüle (10) durch das Betätigungskabel (50, 50', 50") von der unverformten Form (A) in die verformte Form (B, B', B'') gebracht werden kann, um die kleine Gelenkhöhle zu erreichen, während der Zwischenabschnitt (17) immer unverformt ist.

2. Vorrichtung nach Anspruch 1, wobei sich ein flexibler Stab (84) von der distalen starren Kante (54) erstreckt, wobei der flexible Stab (84) eine Vielzahl von Ringabschnitten (85, 86) umfasst, die den flexiblen distalen Abschnitt (15) der Kanüle (10) umgeben, wobei die Vielzahl von Ringabschnitten (85, 86) einen Abschlussring (86) umfasst, mit dem das distale Ende (55) des Betätigungskabels (50) verbunden ist, auf eine solche Weise, dass sich durch Ziehen des Betätigungskabels (50) der flexible Stab (84) biegt und die Ringabschnitte (85, 86) dem flexiblen distalen Abschnitt (15) der Kanüle (10) folgen und ihn von der unverformten Form (A) in die verformte Form (B) führen, wobei das Betätigungskabel (50) angeordnet ist, um in den Ringabschnitten (85, 86) zu gleiten.

3. Vorrichtung nach Anspruch 2, wobei der distale Abschnitt (15) und der Zwischenabschnitt (17) der Kanüle (10) Folgendes umfassen:
- ein gemeinsames röhrenförmiges Kernelement (18), das aus einem flexiblen Material gefertigt ist, wobei das röhrenförmige Kernelement (18) den Extrusionskanal (11) definiert;
- ein gemeinsames Schalenelement (14), welches das röhrenförmige Kernelement (18) umschließt,
wobei das Schalenelement (14) die distale starre Kante (54) und den flexiblen Stab (84) umfasst.

4. Vorrichtung nach Anspruch 3, wobei der flexible Stab (84) einen länglichen Stützabschnitt (87) umfasst, der sich parallel zu einer Längsachse der Kanüle (10) von einem Sektor der distalen Kante (54) erstreckt, wobei sich die Ringabschnitte (85, 86) quer zu dem länglichen Stützabschnitt (87) zwischen zwei gegenüberliegenden lateralen Seiten (88, 89) des länglichen Stützabschnittes (87) erstrecken.

5. Vorrichtung nach Anspruch 1, wobei der flexible distale Abschnitt (15) der Kanüle (10) elastisch nachgiebig ist, wodurch durch Loslassen des Betätigungskabels (50) nach dessen Dehnung der flexible distale Abschnitt (15) aus der verformten Form (B) in die unverformte Form (A) zurückkehrt.

6. Vorrichtung nach Anspruch 1, wobei das zumindest eine Betätigungskabel ein erstes Betätigungskabel (50') ist und ein zweites Betätigungskabel (50") bereitgestellt ist, das konfiguriert ist, um den flexiblen distalen Abschnitt (15) in eine entgegengesetzte Richtung in Bezug auf das erste Betätigungskabel (50') zu ziehen, sodass das erste Betätigungskabel (50') und das zweite Betätigungskabel (50") selektiv als ein Agonisten-Betätigungskabel (50', 50") und ein Antagonisten-Betätigungskabel (50", 50') und umgekehrt funktionieren können.

7. Vorrichtung nach Anspruch 1, wobei zumindest ein weiteres Betätigungskabel (51', 51") bereitgestellt ist, das ein distales Ende umfasst, das mit dem flexiblen distalen Abschnitt (15) an der Extrusionsmündung (16) verbunden und konfiguriert ist, um den flexiblen distalen Abschnitt (15) zu ziehen und ihn aus der unverformten Form (A) in eine weitere verformte Form in einer Ebene zu bringen, die sich von einer Ebene der verformten Form (B, B', B") unterscheidet und durch eine Achse der Kanüle (10) und durch eine Achse des Betätigungskabels (50) definiert ist.

8. Vorrichtung nach Anspruch 1, wobei der Extrusionskanal (11) auf eine Weise konfiguriert ist, die aus der Gruppe ausgewählt ist, umfassend:
- einen zentralen Extrusionskanal (12) und einen ringförmigen Extrusionskanal (13), die koaxial zueinander sind, und wobei der proximale Abschnitt (19) konfiguriert ist, um eine erste therapeutische Substanz in den zentralen Extrusionskanal (12) und eine zweite therapeutische Substanz in den ringförmigen Extrusionskanal (13) zuzuführen und zu drücken und um die therapeutischen Substanzen durch die Extrusionsmündung (16) mit dem flexiblen distalen Abschnitt (15) in der verformten Konformation (B, B', B") abzugeben;
- einen zentralen Extrusionskanal (12), einen ersten ringförmigen Extrusionskanal (13') und einen zweiten ringförmigen Extrusionskanal (13"), die koaxial zueinander sind, und wobei der proximale Abschnitt (19) der Kanüle (10) konfiguriert ist, um eine erste therapeutische Substanz in den ersten ringförmigen Extrusionskanal (13') und eine zweite therapeutische Substanz in den zweiten ringförmigen Extrusionskanal (13") oder eine erste therapeutische Substanz in den zentralen Extrusionskanal (12), eine zweite therapeutische Substanz in den ersten ringförmigen Extrusionskanal (13') und eine dritte therapeutische Substanz in den zweiten ringförmigen Extrusionskanal (13") zuzuführen und zu drücken und um die therapeutischen Substanzen durch die Extrusionsmündung (16) mit dem flexiblen distalen Abschnitt (15) in der verformten Konformation (B, B', B") abzugeben;
- einen ersten und einen zweiten Extrusionskanal (11', 11"), die parallel zueinander sind, und wobei der proximale Abschnitt (19) konfiguriert ist, um eine erste und eine zweite therapeutische Substanz in den ersten bzw. zweiten Extrusionskanal (11', 11") der parallelen Extrusionskanäle zuzuführen und zu drücken und um die therapeutischen Substanzen durch die Extrusionsmündung (16) mit dem flexiblen distalen Abschnitt (15) in der verformten Konformation (B, B', B") abzugeben.

9. Vorrichtung nach Anspruch 8, wobei an der Extrusionsmündung (16) ein starres Abstandselement (71) bereitgestellt ist, an dem das distale Ende (55) des Betätigungskabels (50) befestigt ist, und wobei das starre Abstandselement (71) konfiguriert ist, um mit der Extrusionsöffnung (16) in Eingriff zu kommen und diese offen zu halten, wenn sich der flexible distale Abschnitt (15) von der unverformten Konformation (A) in die verformte Konformation (B, B', B") bewegt.

10. Vorrichtung nach Anspruch 9, wobei das starre Abstandselement (71) eine im Wesentlichen kammartige Form aufweist und einen Stützstab (72) und eine Vielzahl von Zähnen (76, 77) umfasst, die von einer gleichen Seite des Stützstabs (72) vorstehen und in den zentralen Extrusionskanal (12) und in den ringförmigen Extrusionskanal (13) oder in den ersten und zweiten ringförmigen Extrusionskanal (13', 13'') eingeführt sind, um einen vorbestimmten Abstand zwischen Trennwänden der Extrusionskanäle (12, 13, 13', 13'') und einer Außenwand des flexiblen distalen Abschnittes (15) aufrechtzuerhalten, wodurch die Extrusionsmündung (16) offen gehalten wird, wenn sich der flexible distale Abschnitt (15) von der unverformten Konformation (A) in die verformte Konformation (B, B') bewegt.

11. Vorrichtung nach Anspruch 9, wobei das Abstandselement (71)
auch ein Paar Eingriffsabschnitte (74), wobei jeder Eingriffsabschnitt mit dem distalen Abschnitt (55) eines jeweiligen Betätigungskabels (50', 50") in Eingriff kommt, sowie zwei jeweilige Zwischenabschnitte (73) zum Verbinden der Stützstab (72) mit den Eingriffsabschnitten (74) umfasst.

12. Vorrichtung nach Anspruch 1, wobei eine Wand des Extrusionskanals (11) an dem flexiblen distalen Abschnitt (15) aus einem Verbundmaterial hergestellt ist, wobei eine Druckfeder in eine polymere röhrenförmige Matrix eingearbeitet ist, insbesondere durch eine Tauchbeschichtungstechnik.

13. Vorrichtung nach Anspruch 1, wobei der flexible distale Abschnitt (15) der Kanüle (10) aus einem wärmeschrumpfenden Material hergestellt ist, um den flexiblen distalen Abschnitt (15) an dem Zwischenabschnitt (17) zu verankern, indem der Zwischenabschnitt (17) in den distalen Abschnitt eingeführt wird und indem ein anschließender vorbestimmter Wärmezyklus durchgeführt wird.

14. Vorrichtung nach Anspruch 1, wobei der flexible distale Abschnitt (15) eine Länge (L‴) eingestellt zwischen 1 mm und 10 mm aufweist und konfiguriert ist, sodass die verformte Form (B, B', B'') die Form eines Bogens aufweist.

15. Vorrichtung nach Anspruch 1, wobei der flexible distale Abschnitt (15) aus einem Material hergestellt ist, das aus der Gruppe ausgewählt ist, die aus Folgendem besteht:
- Silikonkautschuk
- thermoplastischen Polymeren, insbesondere Polycarbonaten, Polysulfonen, Polyolefinen, insbesondere Polystyrol, Polyethylen oder Polypropylen, Polyurethanen, PVC, Polyestern, insbesondere Polymilchsäure, Polyglykolsäure (PGA), Polyethylenterephthalat (PET), Polyacrylaten, insbesondere Polymethylmethacrylat, Nylon;
- synthetischen Elastomeren, insbesondere Styrol-Butadien- und Styrol-Butadien-Styrol- und Polyester-Kautschuken;
- Naturkautschuk, insbesondere Latex (und Derivaten davon).

## Revendications

1. Dispositif permettant l'administration endoscopique d'au moins une substance thérapeutique dans une petite cavité articulaire, ledit dispositif comprenant :
- une canule (10) définissant au moins un canal d'extrusion (11) et comprenant :
- une partie proximale (19) ;
- une partie distale souple (15) comportant une embouchure d'extrusion (16) et
- une partie intermédiaire sensiblement rigide (17) entre ladite partie distale souple (15) et ladite partie proximale (19),
ladite partie proximale (19) étant conçue pour alimenter et pousser une substance thérapeutique dans ledit canal d'extrusion (11), de sorte que ladite substance thérapeutique puisse passer à travers ledit canal d'extrusion (11) en s'écoulant à travers ladite partie intermédiaire (17) et à travers ladite partie distale souple (15), et puisse ensuite sortir de ladite embouchure d'extrusion (16), comprenant en outre :
- au moins un câble d'actionnement (50,50',50") comportant :
- une extrémité proximale (59) pouvant être actionnée au niveau de ladite partie proximale (19) de ladite canule (10) ;
- une extrémité distale (55) reliée à ladite partie distale souple (15) de ladite canule (10) au niveau de ladite embouchure d'extrusion (16), et conçue pour tirer ladite partie distale souple (15) avec une force transversale audit canal d'extrusion (11), en amenant ladite partie distale souple (15) d'une conformation non déformée (A) à une conformation déformée (B, B', B"),
**caractérisé en ce que**
ladite partie intermédiaire sensiblement rigide (17,57) comporte un bord rigide distal (54) à partir duquel ladite partie distale souple (15) fait saillie, et ledit bord rigide distal (54) comportant une sortie de voie de passage (53) d'une voie de passage (56) pour ledit câble d'actionnement (50,50',50"), ledit bord rigide distal (54) et ladite sortie de voie de passage (53) dudit voie de passage étant disposés de sorte que seule ladite partie distale souple (15) de ladite canule (10) puisse être amené, par ledit câble d'actionnement (50,50',50"), de ladite conformation non déformée (A) à ladite conformation déformée (B,B',B"), afin d'atteindre ladite petite cavité articulaire, tandis que ladite partie intermédiaire (17) est toujours non déformée.

2. Dispositif selon la revendication 1, une tige souple (84) s'étendant depuis ledit bord rigide distal (54), ladite tige souple (84) comprenant une pluralité de parties annulaires (85,86) qui entourent ladite partie distale souple (15) de ladite canule (10), ladite pluralité de parties annulaires (85,86) comprenant un anneau terminal (86) auquel ladite extrémité distale (55) dudit câble d'actionnement (50) est reliée, de manière à ce que, en tirant ledit câble d'actionnement (50), ladite tige souple (84) se plie et lesdites parties annulaires (85,86) suivent et guident ladite partie distale souple (15) de ladite canule (10) de ladite conformation non déformée (A) à ladite conformation déformée (B), ledit câble d'actionnement (50) étant agencé pour coulisser dans lesdites parties annulaires (85, 86).

3. Dispositif selon la revendication 2, ladite partie distale (15) et ladite partie intermédiaire (17) de ladite canule (10) comprenant :
- un élément central tubulaire commun (18) constitué d'un matériau souple, ledit élément central tubulaire (18) définissant ledit canal d'extrusion (11) ;
- un élément de coque commun (14) entourant ledit élément central tubulaire (18),
ledit élément de coque (14) comprenant ledit bord rigide distal (54) et ladite tige souple (84).

4. Dispositif selon la revendication 3, ladite tige souple (84) comprenant une partie support allongée (87) s'étendant parallèlement à un axe longitudinal de ladite canule (10) à partir d'un secteur dudit bord distal (54), lesdites parties annulaires (85,86) s'étendant transversalement à ladite partie support allongée (87) entre deux côtés latéraux opposés (88, 89) de ladite partie support allongée (87).

5. Dispositif selon la revendication 1, ladite partie distale souple (15) de ladite canule (10) étant élastiquement flexible, de sorte que, en libérant ledit câble d'actionnement (50) après son étirement, ladite partie distale souple (15) revienne de ladite conformation déformée (B) à ladite conformation non déformée (A).

6. Dispositif selon la revendication 1, ledit au moins un câble d'actionnement étant un premier câble d'actionnement (50'), et un second câble d'actionnement (50") étant pourvu, conçu pour tirer ladite partie distale souple (15) dans une direction opposée par rapport audit premier câble d'actionnement (50'), de sorte que ledit premier câble d'actionnement (50') et ledit second câble d'actionnement (50") puissent fonctionner de manière sélective en tant que câble d'actionnement agoniste (50',50) et que câble d'actionnement antagoniste (50",50'), et vice-versa.

7. Dispositif selon la revendication 1, au moins un autre câble d'actionnement (51',51") étant pourvu, comprenant une extrémité distale reliée à ladite partie distale souple (15) au niveau de ladite embouchure d'extrusion (16), et conçu pour tirer ladite partie distale souple (15) en l'amenant de ladite conformation non déformée (A) à une autre conformation déformée dans un plan qui est distinct d'un plan de ladite conformation déformée (B,B',B") et est défini par un axe de ladite canule (10), et par un axe dudit câble d'actionnement (50).

8. Dispositif selon la revendication 1, ledit canal d'extrusion (11) étant conçu d'une manière choisie dans le groupe constitué par :
- un canal d'extrusion central (12) et un canal d'extrusion annulaire (13) coaxiaux l'un par rapport à l'autre, et ladite partie proximale (19) étant conçue pour alimenter et pousser une première substance thérapeutique dans ledit canal d'extrusion central (12) et une deuxième substance thérapeutique dans ledit canal d'extrusion annulaire (13), et pour administrer lesdites substances thérapeutiques à travers ladite embouchure d'extrusion (16) avec ladite partie distale souple (15) dans ladite conformation déformée (B,B',B") ;
- un canal d'extrusion central (12), un premier canal d'extrusion annulaire (13') et un second canal d'extrusion annulaire (13") coaxiaux l'un par rapport à l'autre, et ladite partie proximale (19) de ladite canule (10) étant conçue pour alimenter et pousser une première substance thérapeutique dans ledit premier canal d'extrusion annulaire (13') et une deuxième substance thérapeutique dans ledit second canal d'extrusion annulaire (13"), ou une première substance thérapeutique dans ledit canal d'extrusion central (12), un deuxième substance thérapeutique dans ledit premier canal d'extrusion annulaire (13') et une troisième substance thérapeutique dans ledit second canal d'extrusion annulaire (13") et pour administrer lesdites substances thérapeutiques à travers ladite embouchure d'extrusion (16) avec ladite partie distale souple (15) dans ladite conformation déformée (B,B',B") ;
- un premier et un second canal d'extrusion (11',11") parallèles l'un à l'autre, et ladite partie proximale (19) étant conçue pour alimenter et pousser une première et une deuxième substance thérapeutique dans lesdits premier et second canaux d'extrusion (11',11"), respectivement, à partir desdits canaux d'extrusion parallèles et pour administrer lesdites substances thérapeutiques à travers ladite embouchure d'extrusion (16) avec ladite partie distale souple (15) dans ladite conformation déformée (B,B',B").

9. Dispositif selon la revendication 8, au niveau de ladite embouchure d'extrusion (16), étant pourvu un élément d'espacement rigide (71), auquel est fixée ladite extrémité distale (55) dudit câble d'actionnement (50), et ledit élément d'espacement rigide (71) étant conçu pour s'engager avec ladite embouchure d'extrusion (16) et la maintenir ouverte lorsque ladite partie distale souple (15) se déplace de ladite conformation non déformée (A) à ladite conformation déformée (B,B',B").

10. Dispositif selon la revendication 9, ledit élément d'espacement rigide (71) comportant une forme sensiblement en forme de peigne et comprenant une tige support (72) et une pluralité de dents (76,77) faisant saillie d'un même côté de ladite tige support (72) et insérée dans ledit canal d'extrusion central (12) et dans ledit canal d'extrusion annulaire (13) ou dans lesdits premier et second canaux d'extrusion annulaires (13',13"), de manière à maintenir une distance prédéfinie entre les parois de séparation desdits canaux d'extrusion (12,13,13',13"), et une paroi externe de ladite partie distale souple (15), ce qui maintient ladite embouchure d'extrusion (16) ouverte lorsque ladite partie distale souple (15) se déplace de ladite conformation non déformée (A) à ladite conformation déformée (B,B').

11. Dispositif selon la revendication 9, ledit élément d'espacement (71) comprenant également une paire de parties d'engagement (74), chacune s'engageant avec ladite extrémité distale (55) d'un respectif câble d'actionnement (50',50"), ainsi que deux parties intermédiaires respectives (73) de raccordement de ladite tige support (72) avec lesdites parties d'engagement (74).

12. Dispositif selon la revendication 1, une paroi dudit canal d'extrusion (11), au niveau de ladite partie distale souple (15), étant réalisée sous forme de matériau composite, un ressort de compression étant incorporé dans une matrice tubulaire polymère, en particulier par une technique de revêtement par trempage.

13. Dispositif selon la revendication 1, ladite partie distale souple (15) de ladite canule (10) étant constituée d'un matériau thermorétractable, de manière à ancrer ladite partie distale souple (15) à ladite partie intermédiaire (17) en introduisant ladite partie intermédiaire (17) dans ladite partie distale et en effectuant un cycle thermique prédéfini ultérieur.

14. Dispositif selon la revendication 1, ladite partie distale souple (15) comportant une longueur (L‴) fixée entre 1 mm et 10 mm, et étant conçue de sorte que ladite conformation déformée (B,B',B") comporte la forme d'un arc.

15. Dispositif selon la revendication 1, ladite partie distale souple (15) étant constituée d'un matériau choisi dans le groupe constitué par :
- un caoutchouc de silicone
- les polymères thermoplastiques, en particulier les polycarbonates, les polysulfones, les polyoléfines, en particulier le polystyrène, le polyéthylène ou le polypropylène, les polyuréthanes, le PVC, les polyesters, en particulier l'acide polylactique, l'acide polyglycolique (PGA), le téréphtalate de polyéthylène (PET), les polyacrylates, en particulier le polyméthacrylate de méthyle, le nylon ;
- les élastomères synthétiques, en particulier les caoutchoucs styrène-butadiène et styrène-butadiène-styrène et polyester ;
- un caoutchouc naturel, en particulier le latex (et ses dérivés).
